# EUROPEAN PATENT SPECIFICATION

(11) **EP 2 032 521 B1**
(45) Date of publication and mention of the grant of the patent: **28.10.2009**
(21) Application number: 07785845.4
(22) Date of filing: 25.06.2007
(51) Int. Cl.: C07C 209/74

(54) **NEW METHOD FOR SALT PREPARATION**
NEUES VERFAHREN ZUR SALZHERSTELLUNG
NOUVEAU PROCÉDÉ POUR LA PRÉPARATION DES SELS

(30) Priority: 27.06.2006 EP 06116134
(43) Date of publication of application: 11.03.2009
(73) Proprietor: Sandoz AG, 4056 Basel (CH)
(72) Inventor: WIESER, Josef, 6403 Polling (AT); LENGAUER, Hannes, 6250 Kundl (AT); KLINGLER, Elfriede, A-6311 Wildschönau-Oberau (AT); PICHLER, Arthur, 6250 Kundl (AT); STURM, Hubert, 6020 Innsbruck (AT)
(74) Representative: Englmeier, Ludwig
(86) International application number: PCT/EP2007/005596
(87) International publication number: WO 2008/000418

(56) References cited:
- WO-A-2005/012285
- ROBERT WEISS, REINHARD ROTH: "Geminal Bis-Onio Substitution at Trigonal Electron-Deficient Carbon" SYNTHESIS, vol. 10, 1987, pages 870-872, XP002417497
- GLENN NORONHA, KHANHLINH T. NGUYEN: "Preparation of a new class of cleavable surfactants based on the silicon-carbon bond" TETRAHEDRON LETTERS, vol. 40, 1999, pages 4935-4938, XP002417498

## Description

### FIELD OF THE INVENTION

The present invention provides a new method for preparation and crystallization of hydrohalides of pharmaceutical compounds or their intermediates. According to the present process hydrohalides may be obtained in a reliable way with good yield and in pure form consisting of a defined crystal structure. The process of the present invention is especially well suitable for industrial use.

### BACKGROUND OF THE INVENTION

Hydrochlorides of pharmaceutical compounds or their intermediates are usually prepared by acidification of a solution of a base or a salt thereof with hydrogen chloride whereby aqueous hydrogen chloride or gaseous hydrogen chloride is used or a solution of HCl in an organic solvent. The preparation of hydrochlorides by addition of aqueous hydrochloric acid is a straightforward process and hydrochloric acid is conveniently used as the 36% (w/w) solution in water. A typical procedure is to dissolve the organic base in a solvent and to add the calculated volume or an excess of concentrated HCl and if crystallization does not occur, it can be induced by progressive additions of an organic solvent like diethyl ether. However salt formation by aqueous hydrochloric acid is often characterized by lower yields due to solubility of the hydrochloride salt in water. Furthermore if anhydrous salt forms are desired, the use of aqueous hydrochloric acid is not feasible in many cases. If water interferes with the formation and isolation of a solid crystalline product, it is possible to use the anhydrous gas from cylinders or HCl gas in an anhydrous aprotic solvent like diethyl ether. But the alternative use of gaseous hydrogen chloride on a large scale gives rise to high equipment costs and typical risks of gas handling.
Very often it is important to produce a hydrochloride in a pure form consisting of a single crystal structure. This is of particular importance for pharmaceutical drug substance because variations in crystal structure may affect the dissolution, manufacturability and stability of a pharmaceutical drug product, specifically in a solid oral dosage form formulation. But the preferential formation of a desired form of a hydrochloride depends on crystallization kinetics and is not easy to control. Sometimes a constant flow of gaseous hydrogen chloride within a certain time is necessary and the temperature has to be kept substantially constant during the gas flow and even during filtering the product, making the process very difficult to handle.
A purpose of the present invention is to provide a suitable method to prepare hydrochlorides, hydrobromides or hydroiodides of pharmaceutical compounds or their intermediates in a reproducible manner and in a pure form consisting of a defined crystal structure.

### SUMMARY OF THE INVENTION

The present invention provides a new method for preparation and crystallization of hydrohalides of pharmaceutical compounds or their intermediates. According to the present process hydrohalides may be obtained in a reliable way with good yield and in pure form consisting of a defined crystal structure. The process of the present invention is especially well suitable for industrial use.
In particular, the present invention relates to a process for the preparation of a crystalline hydrohalide of an organic amine wherein a trialkylsilylhalogenide is added to the organic amine in a solvent, which organic amine is in the form of the free base or an acid addition salt, wherein the conjugated acid of the acid addition salt is weaker than the hydrohalogenic acid.
It further relates to a Moxifloxacin hydrochloride methylene dichloride solvate, a new anhydrous form IV of Moxifloxacin hydrochloride, a Moxifloxacin hydrochloride nitromethane solvate, a Moxifloxacin hydrochloride acetic acid solvate, pharmaceutical compositions comprising an effective amount of the anhydrous form IV of Moxifloxacin HCl or of the acetic acid solvate of Moxifloxacin HCl, a new crystalline form of Linezolid hydrochloride, a Prasugrel hydrochloride acetonitrile solvate and a Raloxifene hydrochloride tetrahydrofuran solvate, all new solvates or salts being obtainable by the process of the invention. The invention further relates to the use of trialkylsilylhalogenide in a process for the preparation of a crystalline hydrohalide of an organic amine.

### BRIEF DESCRIPTION OF THE DRAWINGS

Figure 1A: X-ray powder diffraction pattern of anhydrous Mycophenolate Mofetil hydrochloride.
Figure 1B: Infrared spectrum of anhydrous Mycophenolate Mofetil hydrochloride.
Figure 1C: DSC curve of anhydrous Mycophenolate Mofetil hydrochloride.
Figure 2A: X-ray powder diffraction pattern of Venlafaxine hydrochloride form I.
Figure 2B: Infrared spectrum of Venlafaxine hydrochloride form I.
Figure 3A: X-ray powder diffraction pattern of Venlafaxine hydrochloride form II.
Figure 3B: Infrared spectrum of Venlafaxine hydrochloride form II.
Figure 4A: X-ray powder diffraction pattern of Sertraline hydrochloride form II.
Figure 4B: Infrared spectrum of Sertraline hydrochloride form II.
Figure 5A: X-ray powder diffraction pattern of Sertraline hydrochloride form I.
Figure 5B: Infrared spectrum of Sertraline hydrochloride form I.
Figure 6A: X-ray powder diffraction pattern of Donepezil hydrochloride form II.
Figure 6B: Infrared spectrum of Donepezil hydrochloride form II.
Figure 7A: X-ray powder diffraction pattern of Donepezil hydrochloride form III.
Figure 7B: Infrared spectrum of Donepezil hydrochloride form III.
Figure 8A: X-ray powder diffraction pattern of Terbinafine hydrochloride.
Figure 8B: Infrared spectrum of Terbinafine hydrochloride form.
Figure 9A: X-ray powder diffraction pattern of Cinacalcet hydrochloride.
Figure 9B: Infrared spectrum of Cinacalcet hydrochloride.
Figure 10A: X-ray powder diffraction pattern of Citalopram hydrobromide.
Figure 10B: Infrared spectrum of Citalopram hydrobromide.
Figure 11A: X-ray powder diffraction pattern of Aripiprazole hydrochloride form A.
Figure 11 B: Infrared spectrum of Aripiprazole hydrochloride form A.
Figure 12A: X-ray powder diffraction pattern of Pramipexole Monohydrochloride.
Figure 12B: Infrared spectrum of Pramipexole Monohydrochloride.
Figure 13A: X-ray powder diffraction pattern of Moxifloxacin hydrochloride methylene dichloride solvate
Figure 13B: Infrared spectrum of Moxifloxacin hydrochloride methylene dichloride solvate.
Figure 13C: ¹H-NMR spectrum (DMSO-d6, TMS) of Moxifloxacin hydrochloride methylene dichloride solvate.
Figure 14A: X-ray powder diffraction pattern of anhydrous Moxifloxacin hydrochloride form IV.
Figure 14B: Infrared spectrum of anhydrous Moxifloxacin hydrochloride form IV.
Figure 15A: X-ray powder diffraction pattern of Moxifloxacin hydrochloride acetic acid solvate.
Figure 15B: Infrared spectrum of Moxifloxacin hydrochloride acetic acid solvate.
Figure 16C: 1H-NMR spectrum (DMSO-d6, TMS) of Moxifloxacin hydrochloride acetic acid solvate.

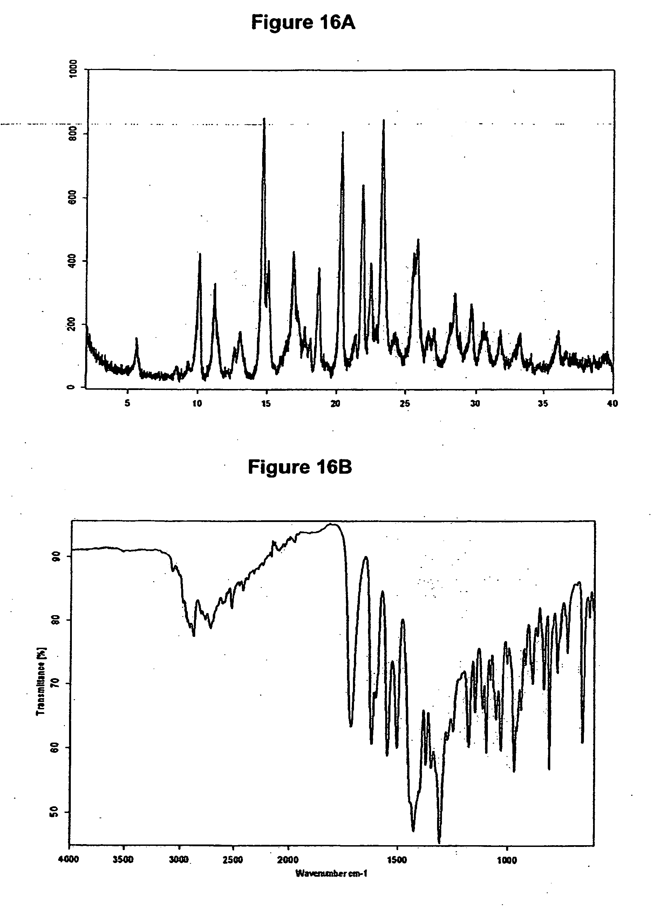

Figure 16A: X-ray powder diffraction pattern of Moxifloxacin hydrochloride nitromethane solvate.
Figure 16B: Infrared spectrum of Moxifloxacin hydrochloride nitromethane solvate.

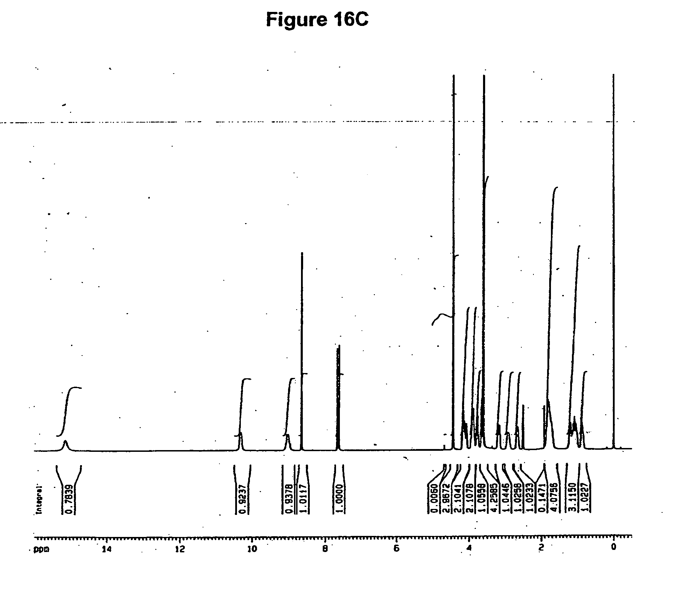

Figure 16C: 1H-NMR spectrum (DMSO-d6, TMS) of Moxifloxacin hydrochloride nitromethane solvate.

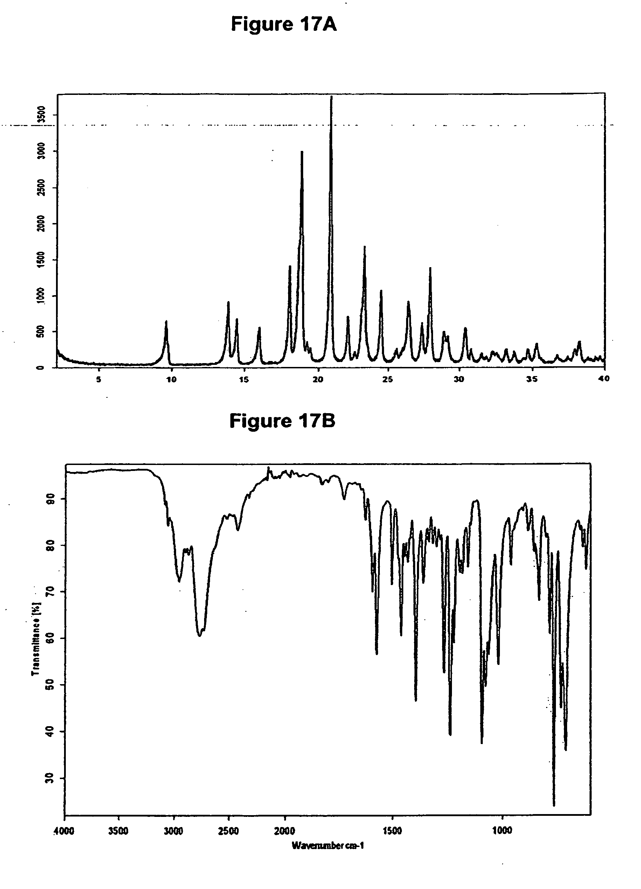

Figure 17A: X-ray powder diffraction pattern of Duloxetine hydrochloride.
Figure 17B: Infrared spectrum of Duloxetine hydrochloride.

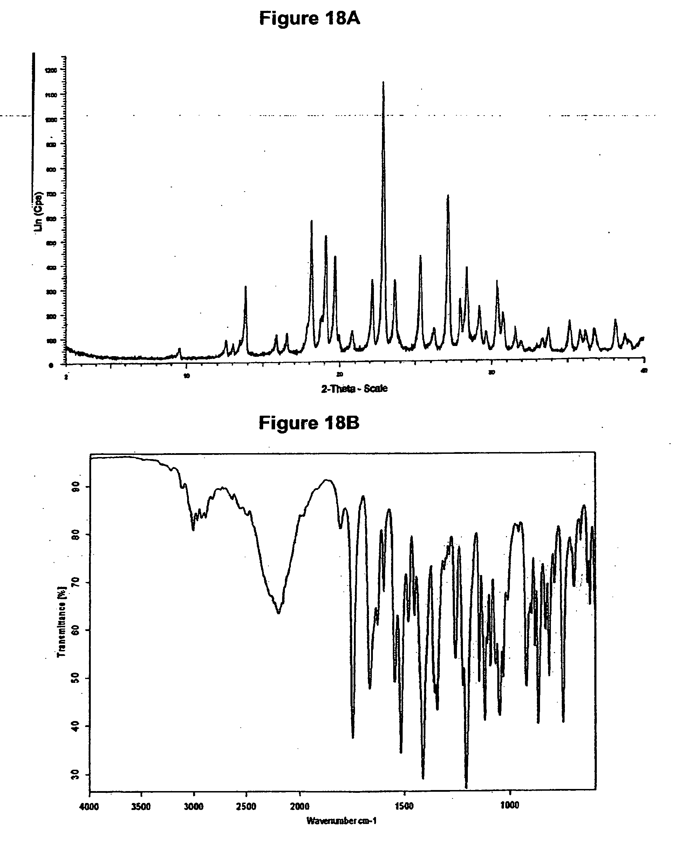

Figure 18A: X-ray powder diffraction pattern of Linezolid hydrochloride.
Figure 18B: Infrared spectrum of Linezolid hydrochloride.

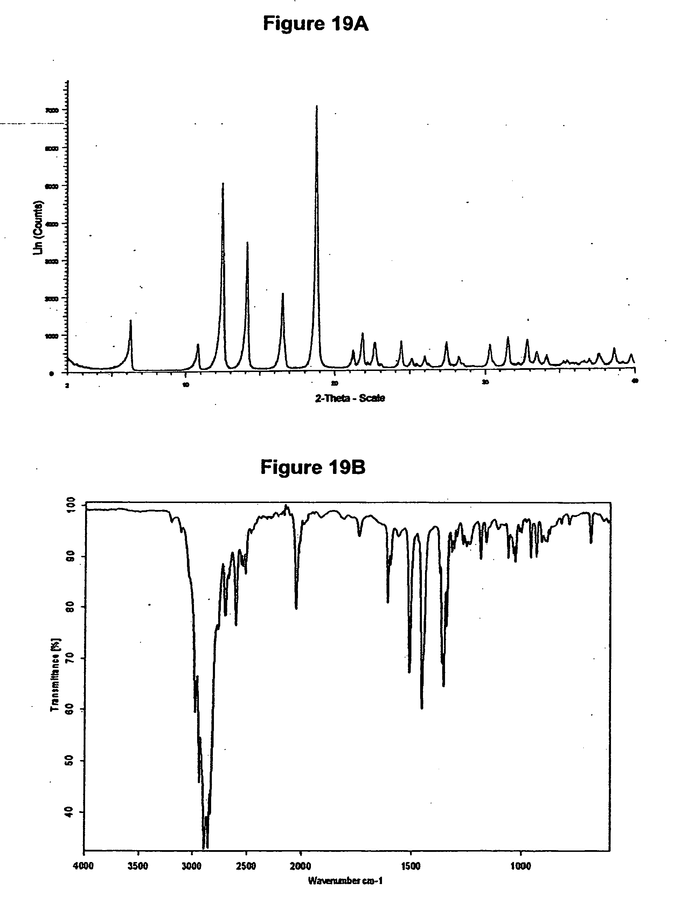

Figure 19A: X-ray powder diffraction pattern of Memantine hydrochloride.
Figure 19B: Infrared spectrum of Memantine hydrochloride.

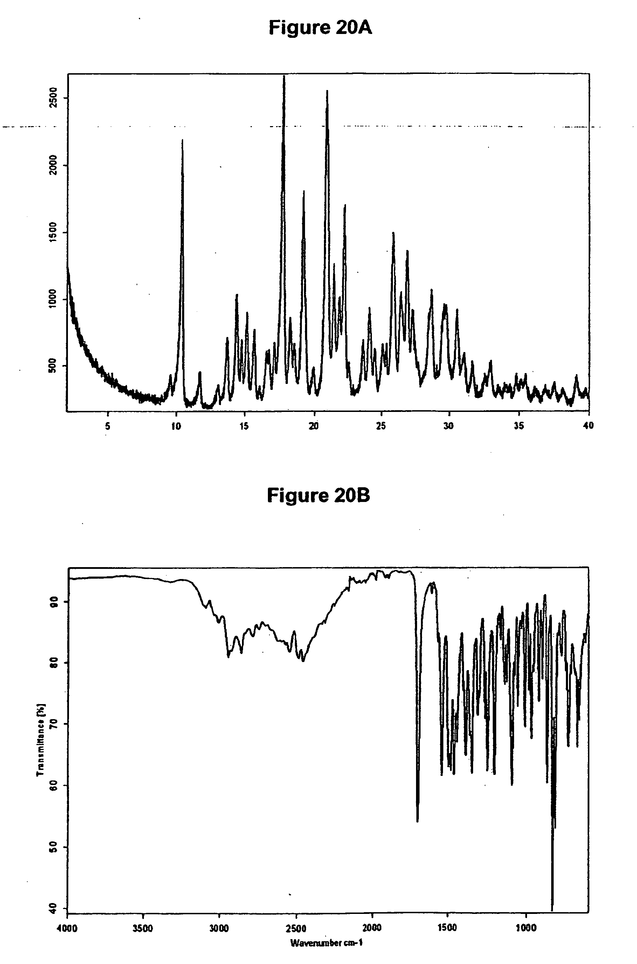

Figure 20A: X-ray powder diffraction pattern of Rimonabant Hydrochloride form I.
Figure 20B: Infrared spectrum of Rimonabant Hydrochloride form I.

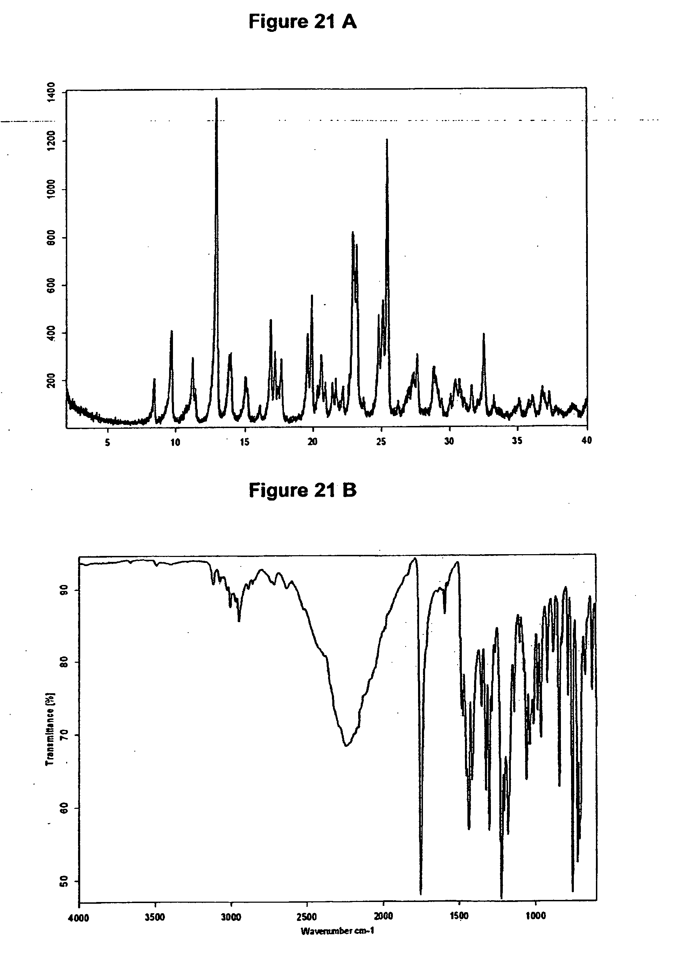

Figure 21 A: X-ray powder diffraction pattern of Clopidogrel Hydrochloride form I.
Figure 21 B: Infrared spectrum of Clopidogrel Hydrochloride form I.

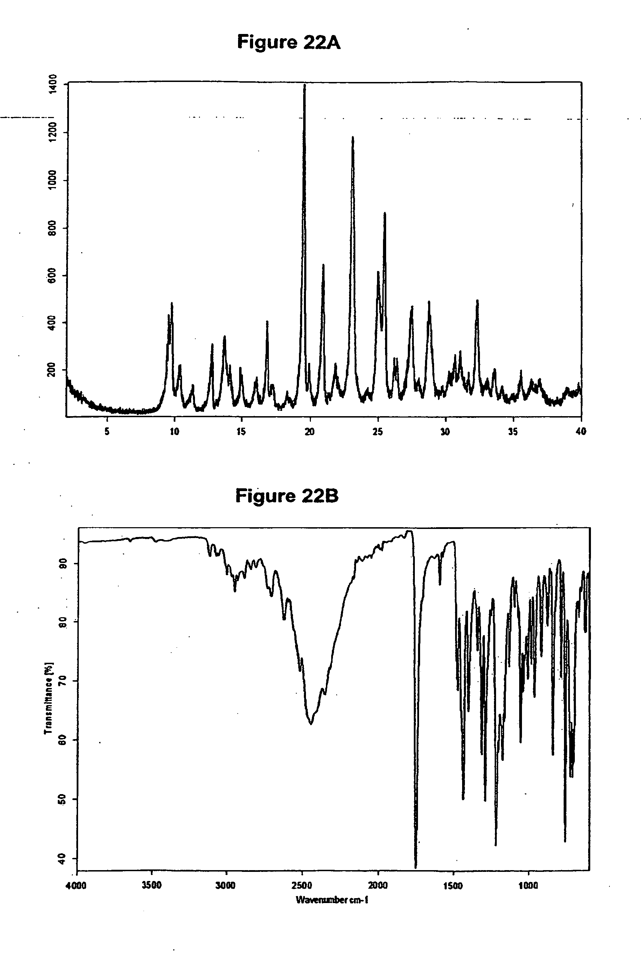

Figure 22A: X-ray powder diffraction pattern of Clopidogrel Hydrobromide form A.
Figure 22B: Infrared spectrum of Clopidogrel Hydrobromide form A.

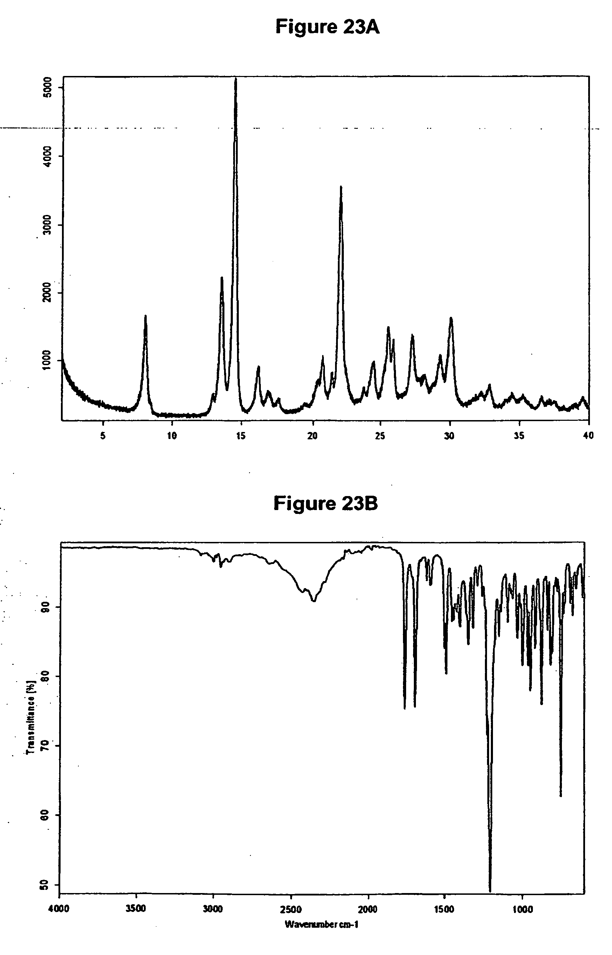

Figure 23A: X-ray powder diffraction pattern of Prasugrel Hydrochloride form B.
Figure 23B: Infrared spectrum of Prasugrel Hydrochloride form B.

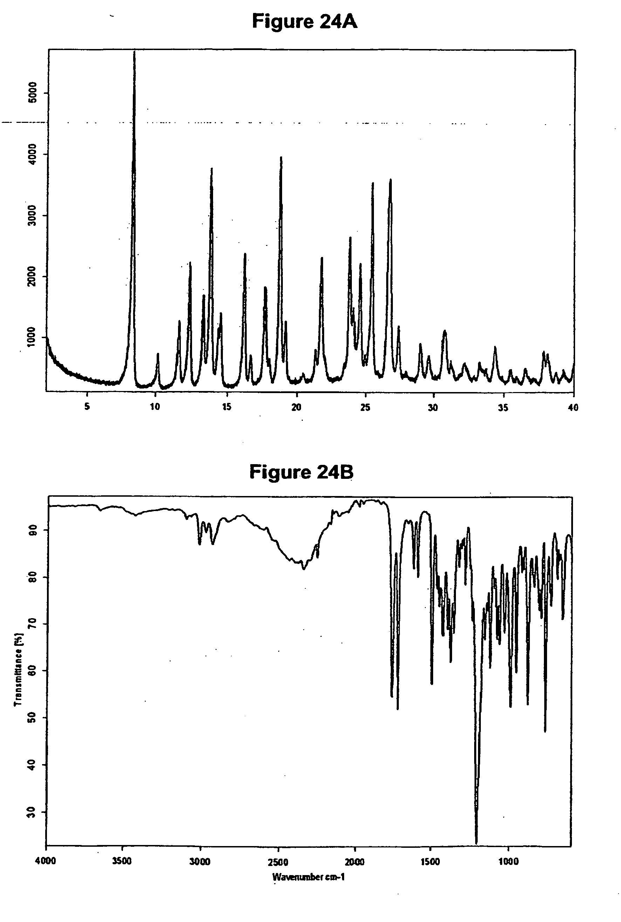

Figure 24A: X-ray powder diffraction pattern of Prasugrel Hydrochloride acetonitrile solvate.
Figure 24B: Infrared spectrum of Prasugrel Hydrochloride acetonitrile solvate.

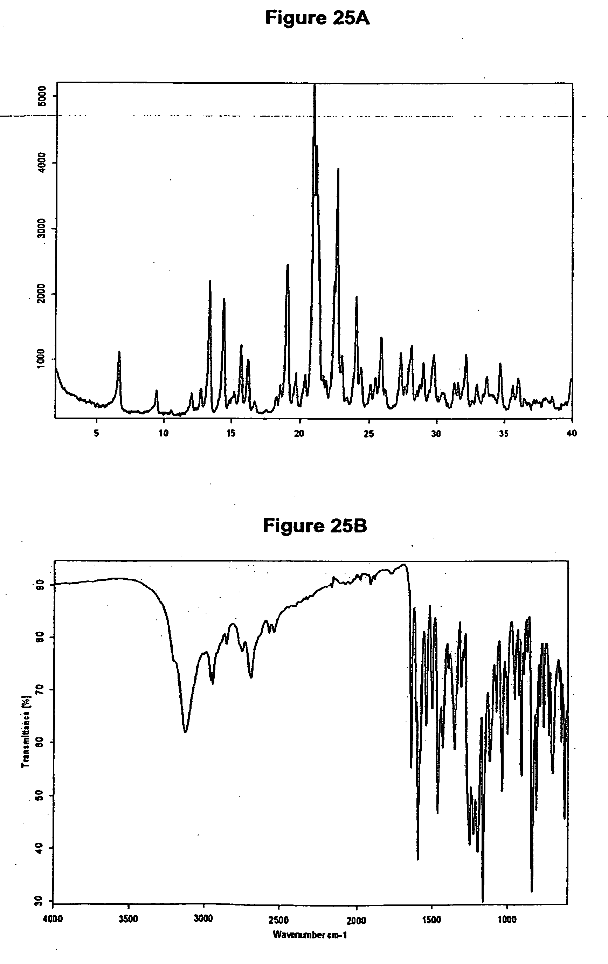

Figure 25A: X-ray powder diffraction pattern of Raloxifene Hydrochloride form A.
Figure 25B: Infrared spectrum of Raloxifene Hydrochloride form A.

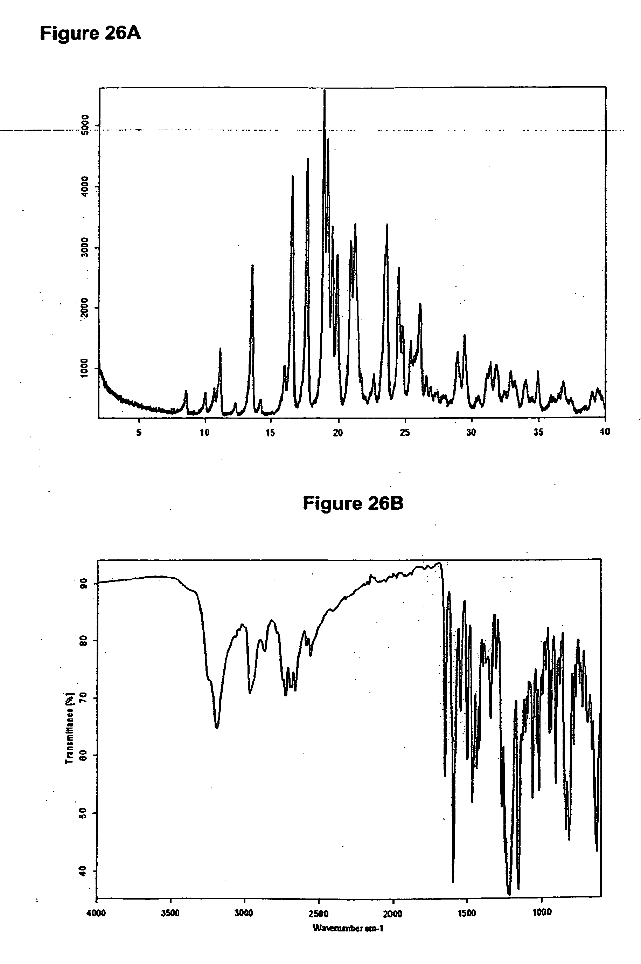

Figure 26A: X-ray powder diffraction pattern of Raloxifene Hydrochloride tetrahydrofurane solvate.
Figure 26B: Infrared spectrum of Raloxifene Hydrochloride tetrahydrofurane solvate.

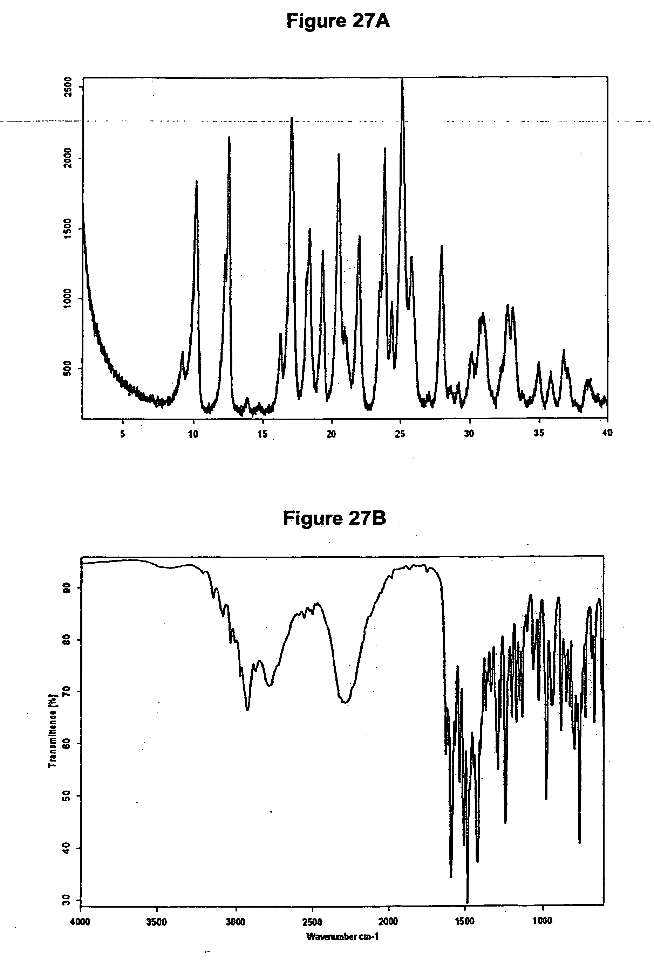

Figure 27A: X-ray powder diffraction pattern of Olanzapine Dihydrochloride form I.
Figure 27B: Infrared spectrum of Olanzapine Dihydrochloride form I.

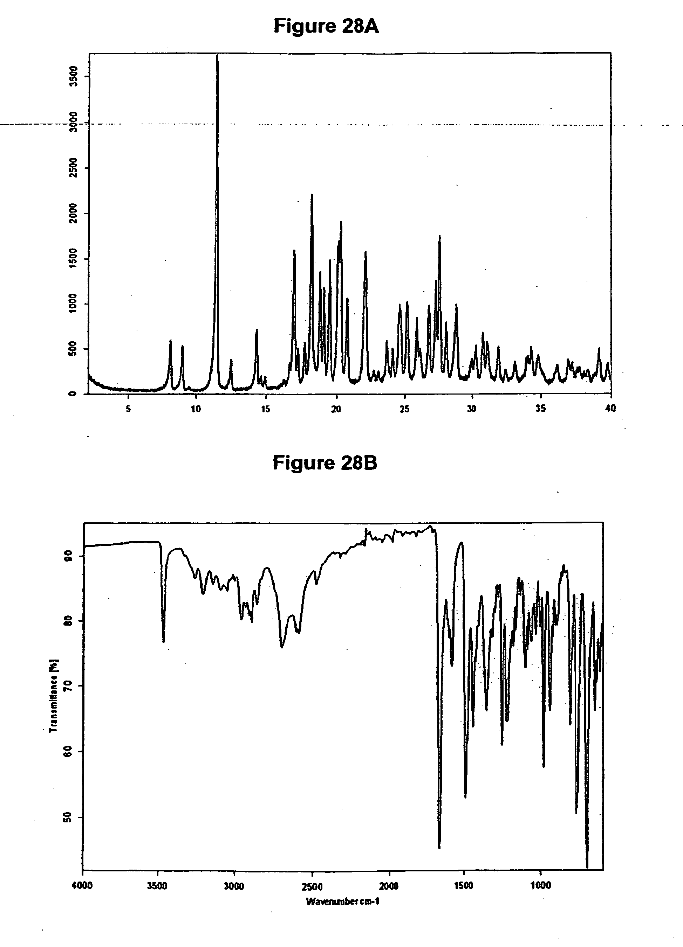

Figure 28A: X-ray powder diffraction pattern of Darifenacin Hydrobromide.
Figure 28B: Infrared spectrum of Darifenacin Hydrobromide. ,

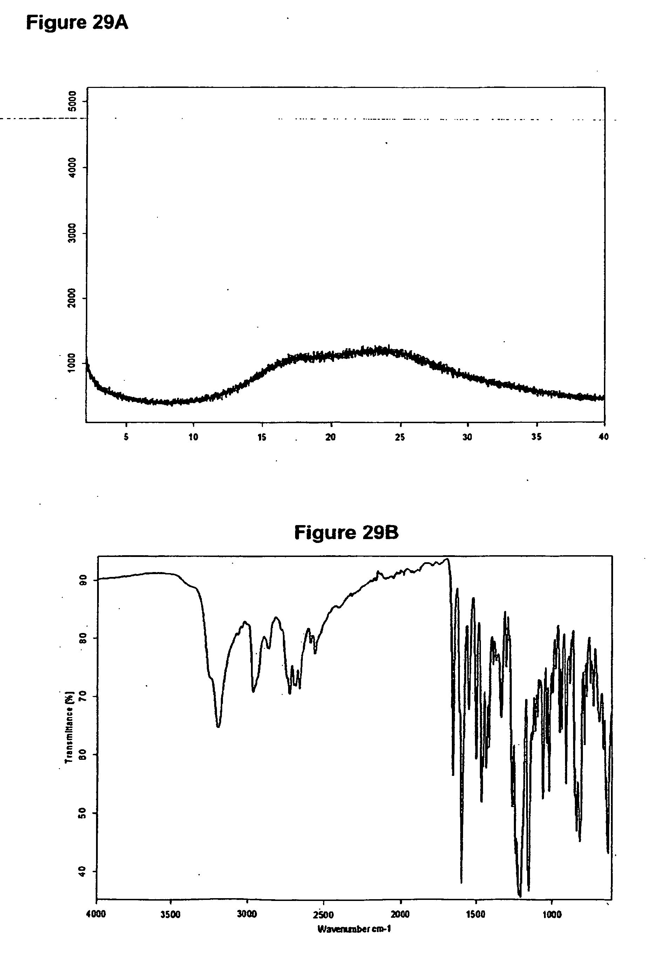

Figure 29A: X-ray powder diffraction pattern of Sitagliptine Hydrochloride in amorphous form.
Figure 29B: Infrared spectrum of Sitagliptine Hydrochloride in amorphous form.

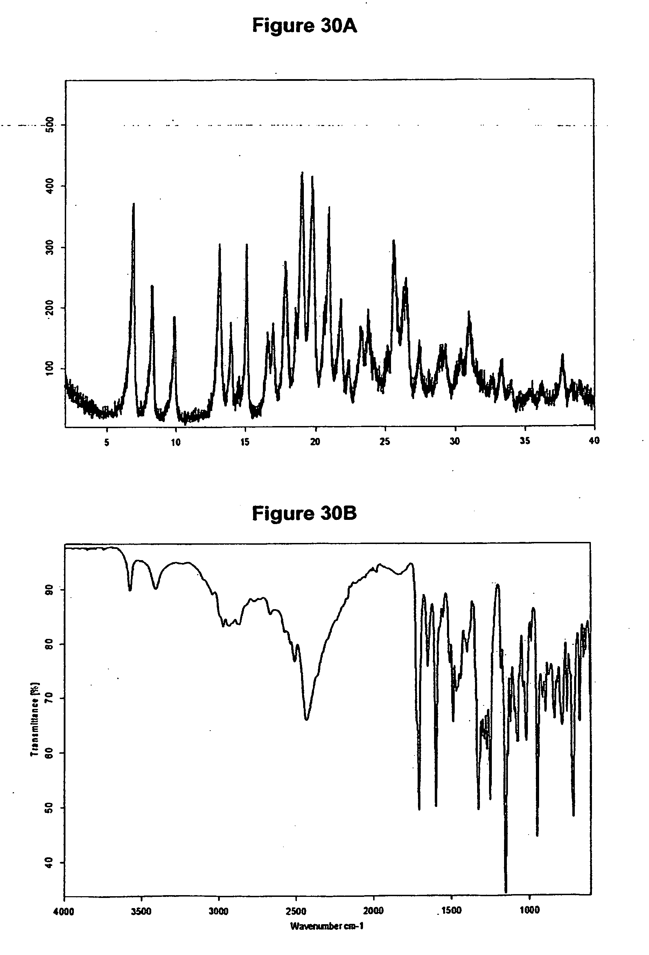

Figure 30A: X-ray powder diffraction pattern of Vardenafil Dihydrochloride.
Figure 30B: Infrared spectrum of Vardenafil Dihydrochloride.

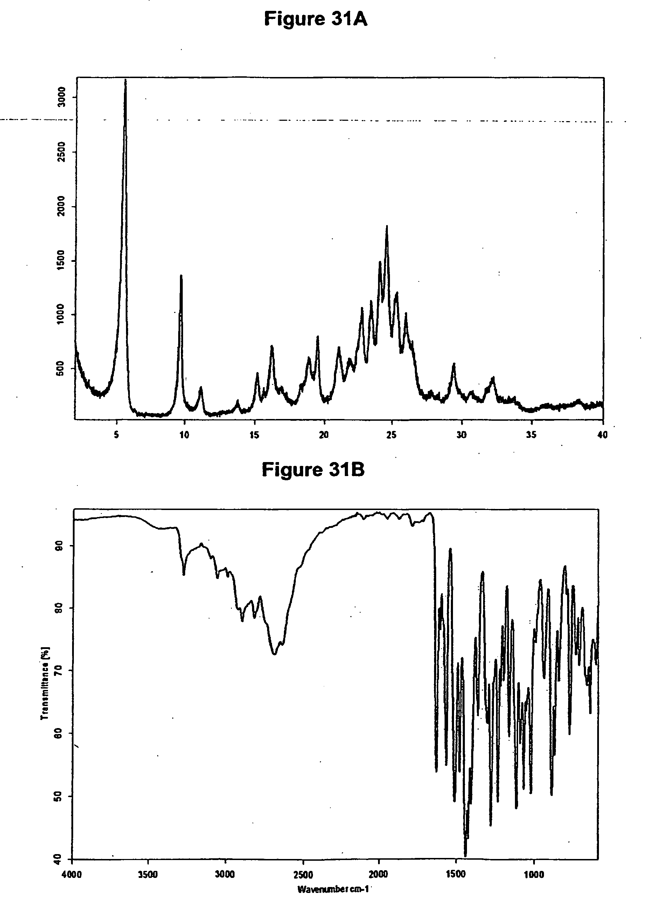

Figure 31 A: X-ray powder diffraction pattern of Erlotinib Hydrochloride form A
Figure 31B: Infrared spectrum of Erlotinib Hydrochloride form A.

### DETAILED DESCRIPTION OF THE INVENTION

The present invention relates to the discovery that trialkylsilylhalogenides are very well suited for the preparation of hydrohalide salts of basic drug substances or hydrohalide salts of basic intermediates, especially in cases where anhydrous conditions are required and/or in cases where a well defined crystalline structure of the salt in pure form has to be prepared.

The invention thus relates to a process for the preparation of a crystalline halogenide of an organic amine wherein a trialkylsilylhalogenide is added to the organic amine in a solvent, which organic amine is in the form of the free base or an acid addition salt, wherein the conjugated acid of the acid addition salt is weaker than the hydrohalogenic acid.

Suitable solvents for the process of the present invention are protic solvents or aprotic solvents in combination with at least one equivalent of a protic solvent compared to the organic amine.
Protic solvents are solvents which are silylable i.e. which have the ability to generate in situ an hydrohalide when added to a trialkylsilylhalogenide. Suitable protic solvents are e.g. aliphatic alcohols or aromatic alcohols, silanols, ketones capable of enolization, or aliphatic or aromatic carbonic acids. Aprotic solvents are solvent which are inert to silylation capable of dissolving or suspending the amine or directing the defined crystal structure of the hydrohalide formed.
Suitable aprotic solvents are e.g. ester, especially ethyl-acetate; nitriles, especially acetonitrile; Ketone, especially acetone; ethers, especially tertiobutylmethylether; Halogenated solvents, especially dichloromethane; Aromatic solvents, especially toluene; Alkanes, especially hexane; and nirtroalkenes, especially nitromethane.
An organic amine within the meaning of the present invention is an organic compound comprising a primary, secondary, tertiary or quaternary amino group and more than three carbon-carbon bonds and having a molecular weight of more than 80Da, preferably having a molecular weight of between 100Da and 5000Da.

In one embodiment the process of the invention comprises the steps of:
(a) dissolving the free base of the organic amine or suspending, preferably with stirring; of the free base of the organic amine in a protic solvent,
(b) adding the trialkylsilylhalogenide,
(c) allowing the crystals of the crystals (hydrohalide of the organic amine) to form and,
(d) collecting the crystals formed.

In preferred embodiments the invention this process is used for the production of moxifloxacin hydrochloride acetic acid solvate, citalopram hydrobromide, clopidrogel hydrobromide, raloxifene hydrochloride form A or erlotinib hydrochloride form A, for example as further detailed below.

During step (b) the halogensilane reacts immediately as silylating agent, presumably with the protic solvent, thereby generating hydrohalogenic acid in situ, and the generated HCl, HBr or HI converts the free base to the hydrogen halide.
Step (d) can be carried out by any suitable method in the art, e.g. by filtration.

Preferred trialkylsilylhalogenides are those where the three alkyl residues are identical, in particular where they are methyl, ethyl, propyl, butyl or isopropyl. Most preferred trialkylhalogenides are trimethylchorosilane, trimethylbromosilane and trimethyliodosilane.

The term "protic solvent" as used in the present invention includes solvent systems which are mixtures of solvents where the total amount of protic solvents is more than one molequivalent compared to the organic amine to be added, in particular wherein the total amount of protic solvents is about one molequivalent of the organic amine to be added.

Preferred protic solvents used in step (a) are solvents comprising a hydroxyl group or a carboxyl group. Particularly preferred protic solvents are aromatic or aliphatic alcohols, silanols, ketones capable of enolization, or aromatic or aliphatic carbonic acids, or solvent systems which are mixtures thereof. More preferred protic solvents are C1-C6 alkyl alcohols, like methanol, ethanol, 2-propanol (isopropanol), butanol (such as n-butanol and isobutanol); or C1-C6 alkylcarboxylic acid, in particular formic acid or acetic acid.
Workup and isolation of the hydrohalides, including drying, may be carried out using methods known in the art.

In a further embodiment, the process for preparing a hydrohalide salt of an organic compound comprises the steps of :
a) dissolving the free base of the organic amine in an aprotic solvent to form a solution or stirring a suspension of the free base in an aprotic solvent,
b) adding at least one equivalent, in particular adding about one equivalent, of a protic solvent to the solution or suspension formed in step a), in particular wherein the preferred protic solvent is as defined above,
c) treating the solution or suspension of step b) with at least one equivalent, in particular with about one equivalent, of a trialkylsilylhalogenide, in particular with a preferred trialkylhalogenide as defined above, to form the corresponding hydrohalide salt,
d) allowing the crystals of the hydrohalide salt of the organic amine to form and,
e) collecting the formed crystals.

Step e) can be carried out by any suitable method in the art, e.g. by filtration.

In preferred embodiments the invention this process is used for, e.g., the production of sertraline hydrochloride form II, aripirazol hydrochloride, pramipexole monohydrochloride, memantine hydrochloride, rimonabant hydrochloride form I, clopidrogel hydrochloride form I, clopidrogel hydrobromide form A, prasugrel hydrochloride form A, prasugrel hydrochloride acetonitrile solvate, raloxifene hydrochloride tetrahydrofuran hemisolvate, olanzapine dihydrochloride form I, darifenacin hydrobromide, sitagliptin hydrochloride or vardenafil dihydrochloride, for example as further detailed below.

When aprotic solvents are used, the free base of the organic amine itself can react with the trialkylhalogenide to form a silylated hydrogen halide and the silylated product can be hydrolyzed afterwards by addition of one or more equivalents of a protic step like an alcohol. Thus, the order of steps b) and c) can be reversed. Also, the trialkylhalogenide can first be mixed with the protic solvent to generate the hydrohalogenic acid and this mixture can then be added to the dissolved or suspended organic amine.

In a further embodiment the process for preparing a hydrohalide salt of an organic compound comprises the steps of:
(a) dissolving, suspending or generating the acid addition salt of the organic amine in a solvent,
(b) the trialkylsilylhalogenide is added,
(c) allowing the crystals of the hydrohalide salt of the organic amine to form and,
(d) collecting the formed crystals.

Step d) can be carried out by any suitable method in the art, e.g. by filtration.

In preferred embodiments the invention this process is used for, e.g., the production of sertraline hydrochloride form II, raloxifene hydrochloride form A or raloxifene hydrochloride tetrahydrofuran hemisolvate, for example as further detailed below.

In step (a) the acid addition salt of the organic amine can be generated in situ by adding the organic acid to the solution or suspension of the base in the solvent
The organic acid used for generation of the organic acid salt of the amine should be weaker than hydrochloric acid and preferably is selected from the group consisting of substituted or unsubstituted alkanoic acids, aromatic carboxylic acids, dicarboxylic acids or citric acid, with acetic acid being particularly preferred.

Preferably in step (a) the acid addition salt of the organic amine is generated in situ by adding an acid, which conjugated acid is weaker than hydrochloric acid and preferably is an organic acid, to a solution or slurry of the organic amine.

In preferred embodiments of the invention this process is used for, e.g., the production of mycophenolate mofetil hydrochloride in its crystalline anhydrous form, venlafaxine hydrochloride form I, venlafaxine hydrochloride form II, sertraline hydrochloride form II, donepezil hydrochloride form III, terbinafine hydrochloride, cinacalcet hydrochloride, moxifloxacin hydrochloride methylene dichloride solvate, moxifloxacine hydrochloride nitromethane solvate, moxifloxacine hydrochloride acetic acid solvate, citalopram hydrobromide, duloxetine hydrochloride or linezolid hydrochloride, for example as further detailed below.

Hydrochlorides have been by far the most frequent choice for salts of basic drugs because of the easy availability and for physiological tolerability, and about half the salts of all drugs are hydrochlorides, and serve as an example for all hydrohalides in the following paragraph.

The formation of hydrochlorides does sometimes require strictly anhydrous conditions and especially in this case it is advantageous to use trialkylsilylchloride for generating hydrochloric acid in situ and for inducing salt formation as described above. At other times formation of hydrochlorides requires a defined stoichiometry of HCl in relation to the organic amine, which is very easy to achieve by use of trialkylsilylchloride as described above. Even if only an exact calculated amount of an hydrohalogenic acid in a solvent is needed, the use of a trialkylsilylhalogenide is easier and a better method than the preparation of the solution via anhydrous gas from cylinders.

In other words, the use of trialkylsilylhalogenides for in situ generation of hydrohalogenic acid in the process of the invention allows a very good control of the stoichiometric ratio of hydrohalogenic acid and organic amine during salt formation. This is of particular advantage when there exist two or more hydrohalides of an organic amine, for example a monohydrohalide and a dihydrohalide, as in this case choosing the amount of hydrohalogenic acid to be generated can direct the process towards obtaining the desired hydrohalide. For example, if one desires to obtain a monohydrohalide, but there exists also a competing dihydrohalide, then one can limit the amount of added trialkylsilylhalogenide so that only one molequivalent of hydrohalogenid acid is generated in situ and thus steer crystallization towards generation of the monohydrohalide.

A further advantage of the process of the invention is that it can also operate at the virtual absence of water so that anhydrous forms of the hydrohalide salts of the organic amines become accessible. Moreover, since the addition of the trialkylsilylhalogenide is a very robust step and can be effected within a broad temperature range and is compatible with a broad diversity of solvent systems, addition of the trialkylsilylhalogenide can most of the times be effected at those very conditions that are known to be optimal for obtaining a desired crystal form. For example, if it is known that polymorphic form A of a monohydrochloride of drug X is obtainable at low temperatures in solvent Y, while polymorphic form B of the same monohydrochloride is obtainable at ambient temperature on solvent Z, then one can simply choose the conditions so that the desired form, be it A or B, forms, for the very reason that the robust nature of the trialkylsilylhalogenide addition step allows to make this choice of crystallization conditions.

In a further embodiment, the process of the invention is employed for the generation of hydrohalides of drugs with a primary, secondary, tertiary or quaternary amino group, in particular wherein the drug is selected from an antidepressant, like the serotonin reuptake inhibitors Sertraline, Duloxetine, Venlafaxine and Citalopram, a nootropic agent, like in particular Donepezil, an antipsychotic agent, like in particular the neuroleptic Aripiprazole and the serotonin/dopamine antagonist Olanzapine, a muscle relaxant, like in particular the antispasmodic agent Memantine, an immunosuppressant, like in particular Mycophenolate Mofetil, an antifungal agent, like in particular Terbinafine, an antibacterial, like in particular a Quinolone, as for example Moxifloxacin or the oxazolidinone Linezolid, a calcimimetic agent, like in particular Cinacalcet, a Dopamine agonist, like in particular the D2-receptor agonist Pramipexole, an antiobesity agent, like Rimonabant, an antithrombotic like Clopidogrel and Prasugrel, an antiosteoporotic, like in particular Raloxifene, an antispasmodic like Darifenacin, an agent for treatment of male erectile dysfunction like Vardenafil, an antidiabetic, like in particular the DPP-IV inhibitor Sitagliptin, an antineoplastic like Erlotinib.

In a further embodiment, the invention relates to the preparation of crystalline anhydrous Mycophenolate Mofetil hydrochloride by a process of the invention, in particular a process which comprises
a) dissolving Mycophenolate Mofetil base in ethyl acetate, acetonitrile or acetone, in particular in ethyl acetate, b) adding acetic acid, in particular 1.0 to about 1.5 equivalents equivalent of acetic acid, and c) treating the solution with a trisilylalkylchlorosilane, in particular with about 1.0 to about 1.5 equivalents of Trimethylchlorosilane.
With this process, anhydrous Mycophenolate Mofetil hydrochloride precipitates and can be isolated in over 97% yield. FT-IR, DSC and XRPD data of the crystalline product correspond to IR, DSC and X-ray crystallography data as shown in WO 95/07902. WO 95/07902 discloses that anhydrous Mycophenolate Mofetil hydrochloride possesses about a two-fold increase in solubility over the monohydrate salt form, while possessing the stability characteristics of the monohydrate salt form. The present method avoids the formation of the monohydrate hydrochloride thus circumventing the need to heat Mycophenolate Mofetil hydrochloride Monohydrate in order to prepare the anhydrous form.

In a further embodiment, the invention relates to the preparation of Venlafaxine hydrochloride by a process of the invention, in particular to a process for the preparation of Venlafaxine hydrochloride Form I or Form II in pure form wherein gaseous hydrogen chloride is not used during the process. In a preferred embodiment, the invention relates to a process for the preparation of Venlafaxine hydrochloride Form I, which process comprises the steps of a) dissolving Venlafaxine base in ethyl acetate, b) adding acetic acid, in particular adding 1.0 to about 1.5 equivalents equivalent of acetic acid, and c) treating the solution with about 1.0 to about 1.5 equivalents of Trimethylchlorosilane. By this process anhydrous Venlafaxine hydrochloride Form I precipitates and can be isolated in over 89% yield. XRPD data of the crystalline product correspond to X-ray crystallography data Form I as shown in WO 02/45658 (Teva) and Form B as shown in WO 02/36542 (Ciba).

In another preferred embodiment, the invention relates to a process for the preparation of Venlafaxine hydrochloride Form II which process comprises the steps of a) dissolving Venlafaxine base in acetone or acetonitrile as solvent, b) adding acetic acid, in particular adding 1.0 to about 1.5 equivalents equivalent of acetic acid, and c) treating the solution with about 1.0 to about 1.5 equivalents of Trimethylchlorosilane. By this process anhydrous Venlafaxine hydrochloride Form II is obtained. XRPD data of the crystalline product correspond to X-ray crystallography data Form II as shown in WO 02/45658 (Teva) and Form C as shown in WO 02/36542 (Ciba).

In a further embodiment, the invention relates to the preparation of Sertraline hydrochloride Form II by a process of the invention. Sertraline hydrochloride Form II is a metastable form and is usually produced by rapid crystallization of Sertraline hydrochloride from an organic solvent. However the preferential formation of form II depends on the rapidity of crystallization which is not easily controllable. Therefore there is a need for the preparation of Sertraline hydrochloride form II. The process of the present invention allows the industrial preparation of this metastable form II in pure form and in a simple way. The present invention therefore also relates to Sertraline hydrochloride form II without detectable levels of sertraline form I, that is less than 1.0% form I, in particular less than 0.5% form I, as determined by the absence of a suitable XRPD peak characteristic for form I alone, for example at 14.9 and 26.3 2 theta.
Therefore, in one embodiment, the invention relates to a process for preparing Sertraline hydrochloride Form II which comprises:
a) dissolving Sertraline free base in an aprotic solvent, for example acetone, methyl ethyl ketone, methyl isobutyl ketone or acetonitrile,
b) adding 1.0 to about 1.5 equivalents equivalent of a protic solvent, like n-butanol or acetic acid, to the solution of step a)
c) treating the solution of step b) with an trialkylsilylchloride, for example Trimethylchorosilane. Trimethylchlorosilane can be added all at once or can be added in two or more portions, or can be added incrementally. The reaction with Trimethylchlorosilane can be conducted at any temperature at which Sertraline is soluble. In acetonitrile as solvent, the reaction is typically conducted at a temperature in the range of from about 20 to 80°C, and more typically at a temperature in the range of from about 20 to 50°C. In methyl isobutyl ketone as solvent the reaction is conducted at a temperature in the range of from about 50 to 100°C, and more typically at a temperature of about 80°C. Trimethylchlorosilane is typically added in an amount of about 1 to about 2 equivalents of Trimethylchlorosilane per equivalent of Sertraline. The protic solvent in step b) is typically added in an amount equivalent to the amount of Trimethylchlorosilane used. Following the addition of Trimethylchlorosilane, the reaction mixture can be aged for a period of time to permit intimate mixing.

In another preferred embodiment the invention relates to a process for preparing Sertraline hydrochloride Form II using an organic salt of Sertraline which comprises
a) stirring a suspension of an organic salt of Sertraline, for example the mandelate or oxalate salt, in an aprotic solvent, for example methyl ethyl ketone, methyl isobutyl ketone or acetonitrile,
b) treating the suspension with a trialkylsilylchloride, for example trimethylchlorosilane.
In methyl ethyl ketone as solvent typically the reaction with Trimethylchlorosilane is conducted at a temperature in the range of from about 50 to 80°C, and in methyl isobutyl ketone as solvent the reaction is conducted at a temperature in the range of from about 50 to 100°C and more, typically at a temperature of about 80°C. In acetonitrile as solvent, the reaction with Trimethylchlorosilane can be conducted even at ambient temperature.
Trimethylchlorosilane is typically added in an amount of from about 1 to about 2 equivalents of Trimethylchlorosilane per equivalent of the organic salt and more typically in an amount of 1,1 equivalents per equivalent of the organic salt.

The present invention further relates to a process for preparing Sertraline hydrochloride Form I which comprises a) dissolving Sertraline free base in isopropanol at room temperature and b) treating the solution with Trimethylchlorosilane.

The present invention further relates to a process of the invention for preparing anhydrous Donepezil hydrochloride. Depending on the choice of solvent, Donepezil hydrochloride Form II or Form III can be made.
In a first embodiment this is a process to prepare Donepezil hydrochloride Form II which comprises:
a) dissolving the Donepezil free base in ethyl acetate, dimethoxyethane or methyl isobutyl ketone,
b) adding at least one equivalent of a protic solvent, for example n-butanol or acetic acid
c) treating the solution with at least one equivalent of Trimethylchlorosilane,
d) isolation of Donepezil hydrochloride Form II
In a second embodiment this is a process to prepare anhydrous Donepezil hydrochloride Form III, wherein acetone or acetonitrile is used as solvent XRPD data of the crystalline product Donepezil hydrochloride Form II and Donepezil hydrochloride Form III correspond to X-ray crystallography data of Form II and Form III as shown in WO 97/46527 (Eisai).

The present invention further relates to a process for preparing Terbinafine hydrochloride, Cinacalcet hydrochloride, Duloxetine hydrochloride, Memantine hydrochloride and Pramipexole Monohydrochloride according to a process of the invention, which process preferably comprises
a) dissolving the free base of Terbinafine, Cinacalcet Duloxetine, Memantine or Pramipexole in an aprotic solvent,
b) adding at least one equivalent of a protic solvent, for example acetic acid or an alcohol like methanol or n-butanol,
c) treating the solution with at least one equivalent of Trimethylchlorosilane.

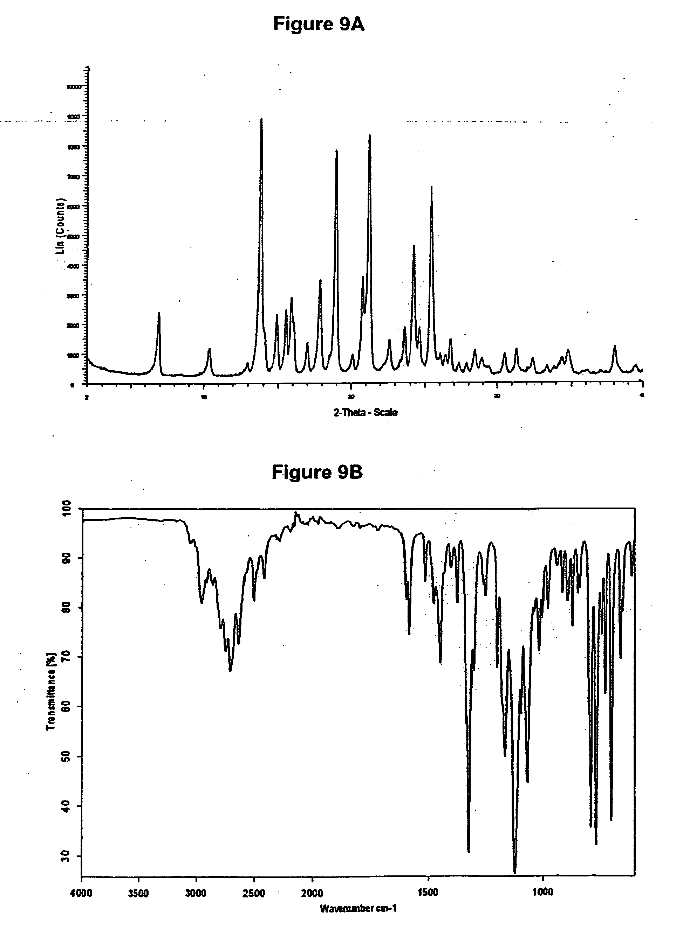

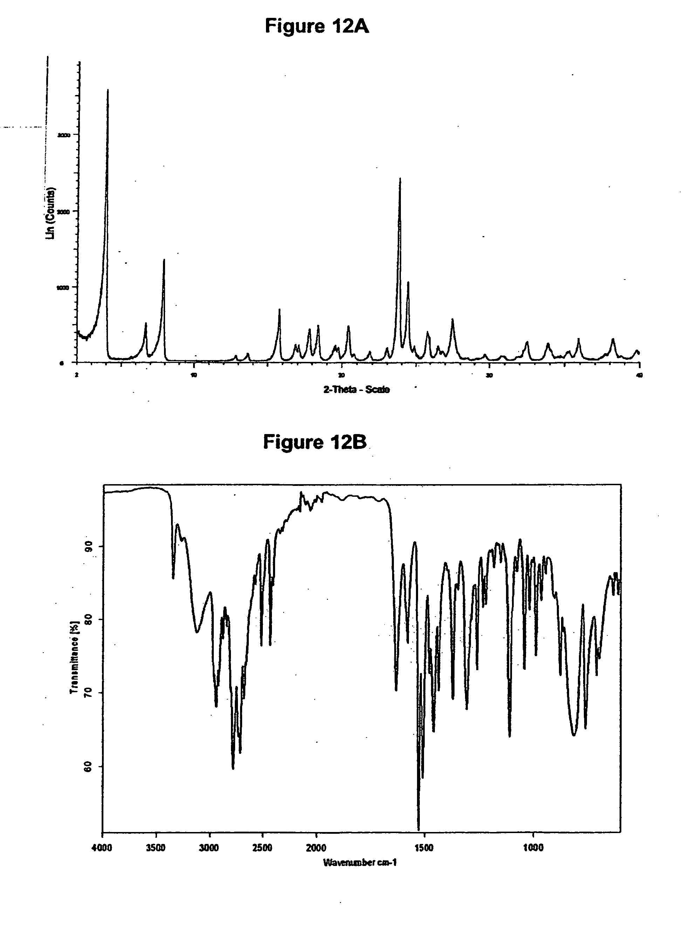

In the above process, Terbinafine can be dissolved for example in an aprotic solvent like acetone, acetonitrile or tert.-butyl methyl ether, Cinacalcet can be dissolved for example in an aprotic solvent like acetonitrile or ethyl acetate, and Pramipexole can be dissolved for example in an aprotic solvent like acetonitrile.
XRPD data of the crystalline product Terbinafine hydrochloride correspond to X-ray crystallography data published by E Tedesco et al. in CrystEngComm, 2002, 4(67), 393-400. A characteristic X-ray powder diffraction pattern of the crystalline hydrochloride salt of Cinacalcet is shown in Figure 9A and a characteristic X-ray powder diffraction pattern of the crystalline hydrochloride salt of Pramipexole is shown in Figure 12A. In addition, the crystalline hydrochloride salt of Cinacalcet is also characterized by a typical infrared spectrum as shown in Figure 9B and the crystalline Pramipexole Monohydrochloride is characterized by a typical infrared spectrum as shown in Figure 12B.
A characteristic X-ray powder diffraction pattern of the crystalline hydrochloride salt of Duloxetine is shown in Figure 18A and a characteristic X-ray powder diffraction pattern of Memantine hydrochloride is shown in Figure 20A. In addition, the crystalline hydrochloride salt of Duloxetine is also characterized by a typical infrared spectrum as shown in Figure 18B and the crystalline Memantine hydrochloride is characterized by a typical infrared spectrum as shown in Figure 19B.

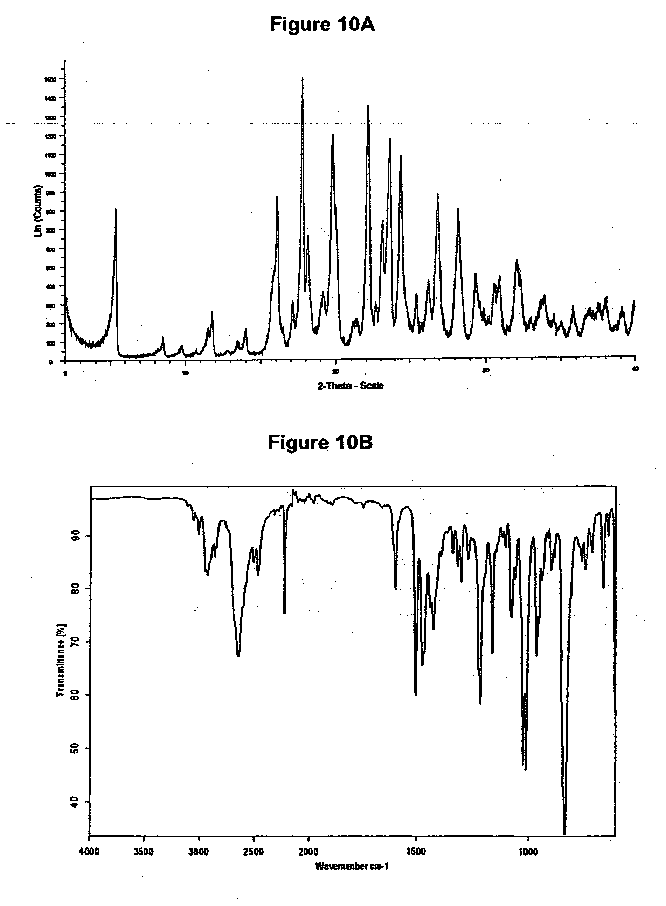

The present invention also relates to a process of the invention for preparing Citalopram hydrobromide by the general process as defined above. In one embodiment the invention relates to a process for preparing Citalopram hydrobromide which comprises dissolving Citalopram free base in a protic solvent like an alcohol i.e. methanol or isopropanol and adding 1.0 to about 1.5 equivalents equivalent Trimethylbromosilane.
In another embodiment the invention relates to a process for preparing Citalopram hydrobromide which comprises:
a) dissolving the free base of Citalopram in an aprotic solvent like ethyl acetate, acetone or acetonitrile,
b) adding at least one, and in particular about one, equivalent of a protic solvent like n-butanol or acetic acid,
c) treating the solution with at least one, and in particular about one, equivalent of Trimethylbromosilane,
d) isolation of Citalopram hydrobromide. Crytalline Citalopram hydrobromide is obtained. A characteristic X-ray powder diffraction pattern of the crystalline Citalopram hydrobromide is shown in
Figure 10A and a typical infrared spectrum is shown in Figure 10B.

The present invention also relates to a process of the invention for preparing Aripiprazole hydrochloride Form A, which process preferably comprises:
a) dissolving the free base of Aripiprazole in an aprotic solvent, for example methylene dichloride,
b) adding at least one, and in particular adding about one, equivalent of a protic solvent like n-butanol or acetic acid,
c) treating the solution with at least one equivalent, and in particular adding about one equivalent, of Trimethylchlorosilane,
d) isolation of Aripiprazole hydrochloride Form A.
XRPD data of the crystalline product Aripiprazole hydrochloride correspond to X-ray crystallography data Form A as shown in WO 2004/083183 (Hetero Drugs Ltd.).

Rimonabant can be suspended for example in acetonitrile or dissolved in ethyl acetate and after addition of at least one equivalent of methanol and Trimethylchlorosilane the Hydrochloride of Rimonabant in crystalline form I is obtained. The X-ray powder diffraction pattern of Rimonabant Hydrochloride form I is shown in Figure 20A. It shows main peaks at 10.4, 14.4, 17.8, 19.2, 20.8, 21.9, 22.2, 26.4, 26.9, 28.7 and 28.5 degrees 2 theta. The infrared spectrum of Rimonabant Hydrochloride form I is shown in Figure 20B. Rimonabant Hydrochloride Form I corresponds to the product prepared according to example 3 of EP 0656354 (Sanofi), in which Rimonabant is dissolved in ether and a saturated solution of HCl gas in ether is added portionwise.

Clopidogrel Hydrochloride in form I can be obtained for example by adding at least one equivalent of acetic acid and trimethylchlorosilane to a solution of Clopidogrel base in ethyl acetate. If acetone or acetonitrile is used as solvent, an anti-solvent like diisopropyl ether is added in order to precipitate the form I of Clopidogrel Hydrochloride. In one preferred embodiment, Clopidogrel base is dissolved in toluene, one equivalent methanol is added and the solution is treated with Trimethylchlorosilane. Following the addition of Trimethylchlorosilane, the fluffy precipitate is stirred for a period of time at room temperature to permit conversion to Clopidogrel Hydrochloride form I.

Prasugrel Hydrochloride form B can be obtained for example by adding at least one equivalent of acetic acid and trimethylchlorosilane to a solution of Prasugrel base in acetone. The XRPD pattern of Prasugrel Hydrochloride form B is shown in Figure 23A and the infrared spectrum is shown in Figure 23B. Characteristic infrared absorption bands are present at 1758 and 1690 cm-1 as reported in US 6693115 in example 3, 4 and 6.

The invention further relates to a novel Prasugrel Hydrochloride acetonitrile solvate, which may be characterized by an X-ray powder diffraction pattern comprising peaks at 8.3, 13.8, 16.2, 18.8, 23.8, 25.4 and 26.8 degrees 2 theta. An example of a X-ray powder diffraction pattern of Prasugrel Hydrochloride acetonitrile solvate is shown in figure 24A. Prasugrel Hydrochloride acetonitrile solvate may be also characterized by a typical infrared spectrum as shown in figure 24B. Characteristic bands are present at 1760, 1720, 1499, 1210 and 775 cm-1. Prasugrel Hydrochloride acetonitrile solvate allows an efficient purification step.

Prasugrel Hydrochloride acetonitrile solvate can preferably prepared by the a process of the present invention comprising the steps of:
a) dissolving Prasugrel in acetonitrile
b) adding at least one equivalent of a protic solvent like acetic acid
c) treating the solution with at least one equivalent of Trimethylchlorosilane
d) isolation of Prasugrel Hydrochloride acetonitrile solvate.

Under air drying conditions, Prasugrel Hydrochloride acetonitrile solvate is stable and does not desolvate. It has an 1 H-NMR spectrum which is substantially identical to the 1 H-NMR spectrum (DMSO-d6, TMS). Specifically, it has a characteristic peak at 2. ppm (s, 3H) which corresponds to about one mol acetonitrile per mol of substance.

Sitagliptin Hydrochloride in amorphous form can be obtained for example by adding at least one equivalent of methanol and trimethylchlorosilane to a solution of Sitagliptin base in a mixture of methylenechloride and diethylether. The XRPD pattern of amorphous Sitagliptin Hydrochloride is shown in Figure 29A and the infrared spectrum is shown in Figure 29B. Characteristic infrared absorption bands are present at and cm-1.

The present invention also relates to a process for preparing Clopidogrel Hydrobromide form A by the general process as defined above which comprises dissolving Clopidogrel free base in a protic solvent like an alcohol i.e. methanol or isopropanol and adding 1.0 to about 1.5 equivalents equivalent Trimethylbromosilane.
Alternatively, the process for preparing Clopidogrel Hydrobromide form A is a process of the invention which comprises:
a) dissolving the free base of Clopidrogel in a non protic solvent like ethyl acetate,
b) adding at least one equivalent of a protic solvent like acetic acid,
c) treating the solution with at least one equivalent of Trimethylbromosilane,
d) isolation of Clopidogrel Hydrobromide form A.
A characteristic X-ray powder diffraction pattern of the crystalline Clopidogrel Hydrobromide is shown in Figure 22A and a typical infrared spectrum is shown in Figure 22B.

The present invention further relates to a process of the present invention for preparing Raloxifene Hydrochloride Form A. In a preferred embodiment the process for preparing Raloxifene Hydrochloride Form A comprises
a) stirring a suspension of Raloxifene free base in a protic solvent like ethanol
b) treating the suspension with Trimethylchorosilane.

Alternatively the process for preparing Raloxifene Hydrochloride Form A comprises
a) stirring a suspension of an organic salt of Raloxifene like the lactate in a protic solvent like ethanol or in a non protic solvent like methyl isobutyl ketone or acetonitrile
b) treating the suspension with Trimethylchlorosilane.
In methyl isobutyl ketone as solvent typically the reaction with Trimethylchlorosilane is conducted at a temperature of about 100°C, in acetonitrile as solvent, the reaction with Trimethylchlorosilane is typically conducted at ambient temperature.
A characteristic X-ray powder diffraction pattern of Raloxifene Hydrochloride Form A is shown in Figure 25A and a typical infrared spectrum is shown in Figure 25B

The present invention also relates to a novel Raloxifene Hydrochloride Hemisolvate with tetrahydrofuran, which may be characterized by an X-ray powder diffraction pattern comprising peaks at 13.6, 16.6, 17.7, 18.9, 19.2, 19.5, 21.2 and 23.6 degrees 2 theta. An example of an X-ray powder diffraction pattern of Raloxifene Hydrochloride Hemisolvate with tetrahydrofuran is shown in figure 26A. Raloxifene Hydrochloride Hemisolvate with tetrahydrofuran may be also characterized by a typical infrared spectrum as shown in figure 26B. Characteristic bands are present at 1651, 1595, 1226, 1165, 838 and 815 cm-1. Raloxifene Hydrochloride Hemisolvate with tetrahydrofuran allows a more efficient and/or effective purification step for raloxifene production.

The invention also relates to a process of the present invention for the preparation of Raloxifene Hydrochloride Hemisolvate with tetrahydrofuran comprising the steps of:
a) stirring a suspension of Raloxifene free base in tetrahydrofuran
b) adding 1.0 to about 1.5 equivalents equivalent of a protic solvent like methanol to the suspension of step a)
c) treating the suspension of step b) with Trimethylchorosilane.
d) isolation of the tetrahydrofuran Hemisolvate of Raloxifene Hydrochloride.

An alternative embodiment of the process for preparing the Raloxifene Hydrochloride Hemisolvate with tetrahydrofuran is a process using an organic salt of Raloxifene which comprises
a) stirring a suspension of an organic salt of Raloxifene like the lactate in tetrahydrofuran
b) treating the suspension with Trimethylchlorosilane.
c) isolation of Raloxifene Hydrochloride Hemisolvate with tetrahydrofuran.
Under air drying conditions, Raloxifene Hydrochloride THF Hemisolvate is stable and does not desolvate. It has an 1H-NMR spectrum which is substantially identical to the 1H-NMR spectrum (DMSO-d6, TMS).

The present invention further relates to a process of the invention for preparing anhydrous Vardenafil Dihydrochloride, which process comprises:
a) dissolving Vardenafil base in an aprotic solvent or for example in a solvent mixture like diethyl ether and methylene dichloride as described in example 10 of EP 1049695
b) adding at least two equivalents of a protic solvent like methanol
c) treating the solution with at least two equivalents of Trimethylchlorosilane.
d) isolation of anhydrous Vardenafil Dihydrochloride
A characteristic X-ray powder diffraction pattern of anhydrous Vardenafil Dihydrochloride is shown in Figure 30A and a typical infrared spectrum is shown in Figure 30B

The present invention further relates to a process of the present invention for preparing Erlotinib Hydrochloride Form A, which process comprises suspending Erlotinib base in isopropanol and treating the suspension with at least one equivalent of Trimethylchlorosilane at room temperature. A characteristic X-ray powder diffraction pattern of Erlotinib Hydrochloride Form A is shown in Figure 31A and a typical infrared spectrum is shown in Figure 31B. XRPD data of the crystalline product Erlotinib Hydrochloride Form A correspond to X-ray crystallography data Form B as shown in US 2004/0162300 (Hofmann-La Roche).

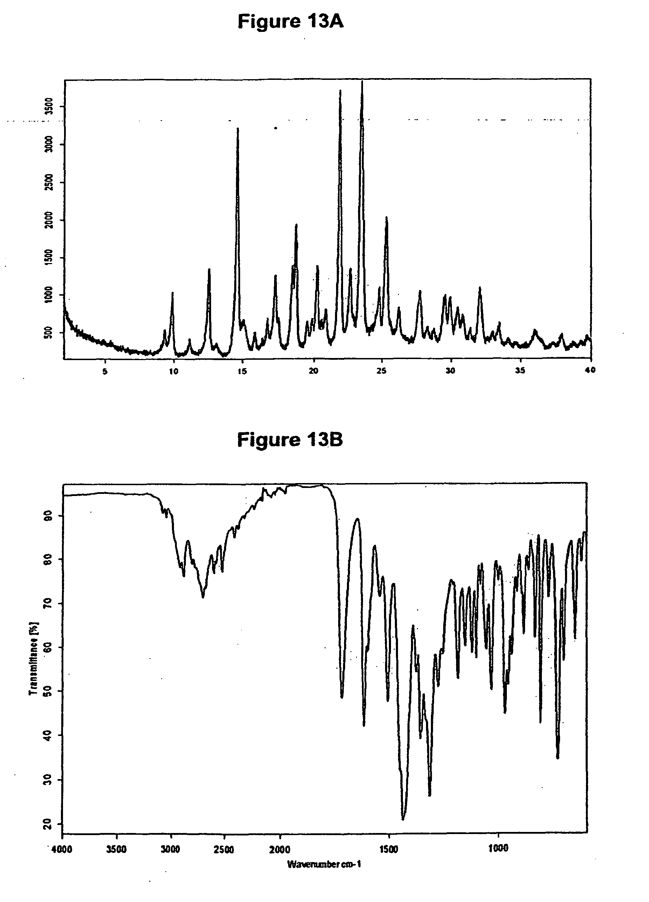

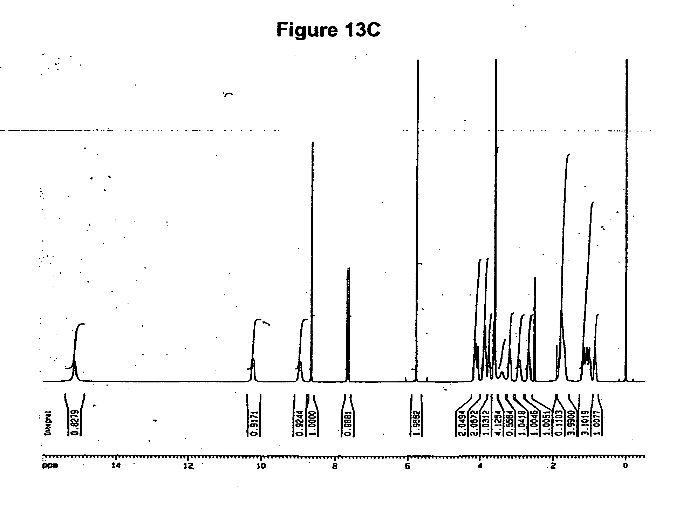

By employing the processes of the invention, we have further discovered a Moxifloxacin hydrochloride methylene dichloride solvate characterized by an X-ray powder diffraction pattern with peaks at about 14.6, 18.7, 21.9, 23.5, and 25.3 degrees 2 theta. The invention therefore also relates to a Moxifloxacin hydrochloride methylene dichloride solvate, in particular wherein the molar ratio of Moxifloxacin hydrochloride to methylene dichloride is about 1:1, in particular this solvate demonstrates peaks in a X-ray powder diffraction pattern at about 14.6, 18.7, 21.9, 23.5, and 25.3 degrees 2 theta. A characteristic X-ray powder diffraction pattern of Moxifloxacin hydrochloride methylene dichloride solvate is shown in figure 13A. Moxifloxacin hydrochloride methylene dichloride solvate for the first time enables preparation of the below-described Moxifloxacin hydrochloride form IV.
Moxifloxacin hydrochloride methylene dichloride solvate may be also characterized by characteristic bands in an infrared spectrum at about 2704, 1720, 1434, 1311, 1272, 730 and 702 cm⁻¹, in particular by the typical infrared spectrum as shown in figure 13B.
Moxifloxacin hydrochloride methylene dichloride solvate may be prepared by the process of the invention comprising the steps of:
a) dissolving Moxifloxacin in methylene dichloride
b) adding at least one equivalent, and in particular adding about one equivalent, of a protic solvent, for example n-butanol or acetic acid
c) treating the solution with at least one equivalent, and in particular adding about one equivalent, of a trialkylchlorosilane, for example Trimethylchlorosilane
d) isolation of Moxifloxacin hydrochloride methylene dichloride solvate.
Under air drying conditions, Moxifloxacin hydrochloride methylene dichloride solvate is stable and does not desolvate. TGA of Moxifloxacin hydrochloride methylene dichloride solvate shows a loss of mass of 16% between 80 and 180°C, which corresponds to one mol dichloromethane per mol of substance. The invention therefore relates to Moxifloxacin hydrochloride methylene dichloride solvate showing a loss of mass of between 10% and 20%, and in particular about 16%, between 80 and 180°C, which corresponds to about one mol dichloromethane per mol of substance.
Moxifloxacin hydrochloride methylene dichloride solvate has an ¹H-NMR spectrum which is substantially identical to the ¹H-NMR spectrum (DMSO-d6, TMS) shown in Figure 13C. Specifically, it has a characteristic peak at 5.77 ppm (s, 2H) which corresponds to about one mol methylene dichloride per mol of substance.

By employing the processes of the invention, we have further discovered a Moxifloxacin hydrochloride nitromethane solvate. The invention therefore also relates to such a solvate, in particular wherein the molar ratio of Moxifloxacin hydrochloride to nitromethane is about 1:1. The solvate can be further characterized by an X-ray powder diffraction pattern with peaks at about 11.2, 14.7, 16.9, 20.4, 22.0, 22.6 and 23.4 degrees 2 theta, in particular by the characteristic X-ray powder diffraction pattern of Moxifloxacin hydrochloride nitromethane solvate as shown in figure 16A. Moxifloxacin hydrochloride nitromethane solvate may also be characterized by characteristic bands in an infrared spectrum at about 2879, 1715, 1550, 1310 and 1032 cm⁻¹, in particular by the typical infrared spectrum as shown in figure 16B. Moxifloxacin hydrochloride nitromethane solvate allows a more efficient and/or effective purification step for moxifloxacin production.
Moxifloxacin hydrochloride nitromethane solvate may be prepared by the process of the invention, in particular comprising the steps of:
a) dissolving Moxifloxacin in nitromethane
b) adding at least one equivalent, and in particular adding about one equivalent, of a protic solvent like n-butanol or acetic acid
c) treating the solution with at least one equivalent, and in particular adding about one equivalent, of Trimethylchlorosilane
d) isolation of Moxifloxacin hydrochloride nitromethane solvate.
Moxifloxacin hydrochloride nitromethane solvate has an ¹H-NMR spectrum which is substantially identical to the ¹H-NMR spectrum (DMSO-d6, TMS) shown in Figure 16D. Specifically, it has a characteristic peak at 4.4 ppm (s, 3H) which corresponds to about one mol nitromethane per mol of substance.

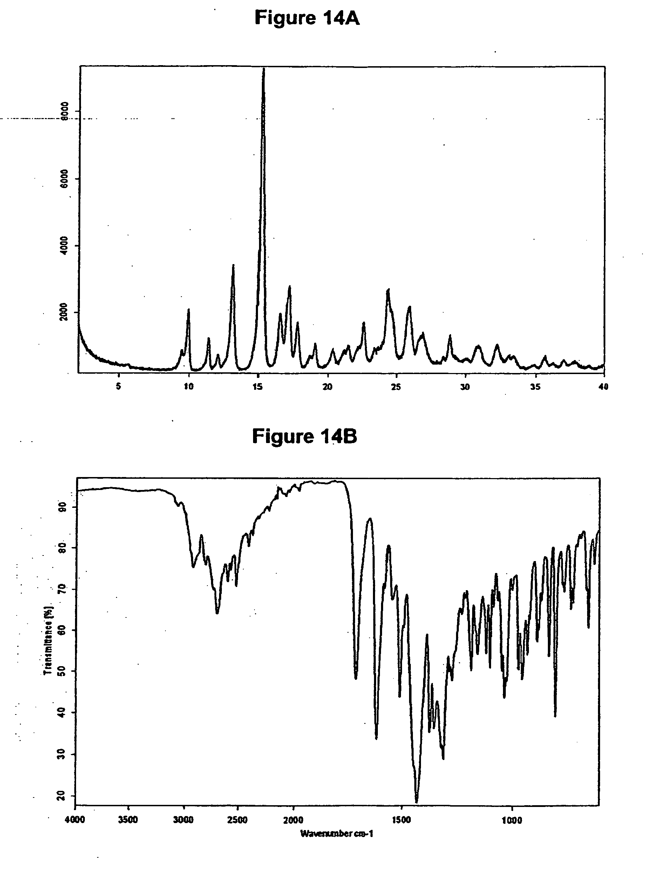

The present invention also relates to a novel anhydrous hydrochloride salt of Moxifloxacin, herein called form IV. In particular, form IV does not convert to a hydrous form of Moxifloxacin hydrochloride, when placed in a dessicator at a humidity level of 33% for 48 hours. More particular, the invention relates to form IV characterized by an X-ray powder diffraction pattern with peaks at about 10.0, 13.2, 15.3, 17.2 and 24.4 degrees 2 theta. A characteristic X-ray powder diffraction pattern of the anhydrous form IV of Moxifloxacin hydrochloride is shown in figure 14A. The invention also relates to form IV characterized by infrared absorption bands at about 2704, 1720, 1434, 1312 and 1273 cm⁻¹. Moxifloxacin hydrochloride form IV may further be characterized by showing a typical infrared spectrum as shown in figure 14B.
The new anhydrous form IV may preferably be prepared by desolvating Moxifloxacin hydrochloride methylene dichloride solvate by drying in vacuum at about 100°C. The anhydrous form IV shows a lower hygroscopicity as anhydrous form II described in US 5849 752 (Bayer) and a lower hygroscopicity than anhydrous form B described in Chemi Spa's patent application WO 2005/054240, facilitating its handling and/or storage. After placing Moxifloxacin hydrochloride anhydrous form IV in a dessicator at a humidity level of 33% for 48 hours anhydrous form IV does not convert to a hydrous form of Moxifloxacin hydrochloride, This is demonstrated by the XRPD pattern which shows no alteration and by the moisture content which rises only from 0.5% to 0.6%. The invention also relates to pharmaceutical compositions comprising form IV.

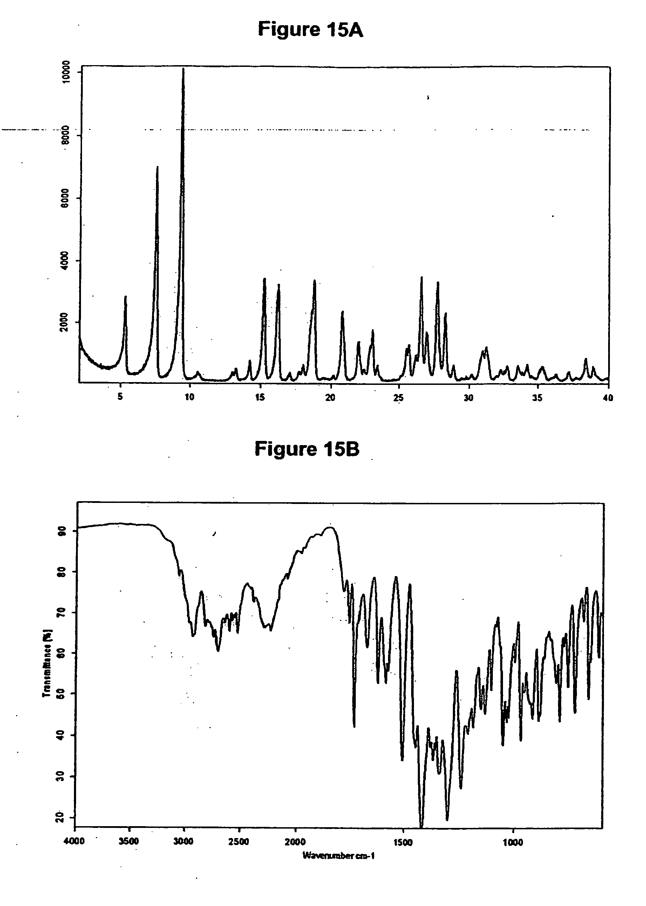

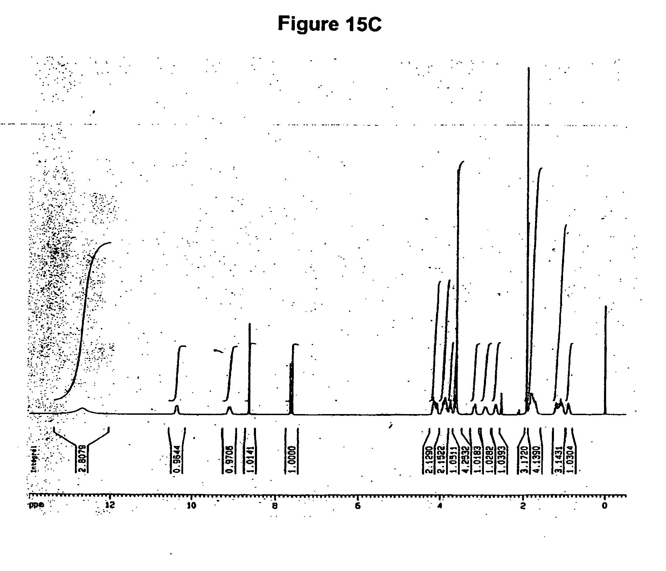

By employing the processes of the invention, we have further discovered a Moxifloxacin hydrochloride acetic acid solvate. The invention therefore also relates to such a solvate, in particular wherein the molar ratio of Moxifloxacin hydrochloride to acetic acid is about 1:1. This solvate may be further characterized by an X-ray powder diffraction pattern with peaks at about 5.3, 7.6, 9.3, 15.2, 16.2, 18.8, 20.8, 26.6 and 27.7 degrees 2 theta. In a preferred embodiment the moxifloxacin hydrochloride acetic acid solvate shows a X-ray powder diffraction pattern substantially in accordance with the one shown in figure 15A. Moxifloxacin hydrochloride acetic acid solvate may also be characterized by a typical infrared spectrum as shown in figure 15B. Exemplary infrared absorption bands of Moxifloxacin hydrochloride acetic acid solvate can be observed at 2707, 2289, 1736, 1421, 1308, 1246, 917 and 757 cm⁻¹. Moxifloxacin hydrochloride acetic acid solvate shows good solubility and/or stability. Moxifloxacin hydrochloride acetic acid solvate may be prepared by the process as defined and described above, which comprises:
a) dissolving Moxifloxacin in acetic acid or in a mixture of acetic acid and an organic solvent like acetone or acetonitrile
b) adding Trimethylchlorosilane
c) isolation of Moxifloxacin hydrochloride acetic acid solvate.
Moxifloxacin hydrochloride acetic acid solvate has an ¹H-NMR spectrum which is substantially identical to the ¹H-NMR spectrum (DMSO-d6, TMS) shown in Figure 15C. Specifically, it has a characteristic peak at 1.9 ppm (s, 3H) which corresponds to about one mol acetic acid per mol of substance. The invention also relates to pharmaceutical compositions comprising the Moxifloxacin hydrochloride acetic acid solvate of the invention.

The present invention also relates to a process of the invention for preparing Linezolid hydrochloride, which preferably comprises adding a protic solvent like n-butanol or acetic acid to a solution of Linezolid in an organic solvent like acetone or acetonitnle and treating the mixture with Trimethylchlorosilane.
The present invention also relates to crystalline Linezolid hydrochloride. Linezolid hydrochloride may be characterized by, for example, characteristic peaks in the XRPD pattern at values of about 13.9, 18.2, 19.1, 23.0 and 27.2 degrees two theta. A characteristic X-ray powder diffraction pattern of the crystalline Linezolid hydrochloride is shown in Figure 19A and a typical infrared spectrum is shown in Figure 19B. As shown in figure 19A, Linezolid hydrochloride shows characteristic peaks in the XRPD pattern at values of about 13.9, 18.2, 19.1, 23.0 and 27.2 degrees two theta. Surprisingly, this hydrochloride has been found to be stable, something which could not have been predicted based on the chemical structure of Linezolid. Linezolid hydrochloride will allow the production of pharmaceutical compositions.

As explained above, the invention allows fine-tuning of the amount of hydrohalogenic acid generated in the crystallization solution, of the speed with which said hydrohalogenic acid is generated - for example by controlling the rate of addition of the trialkylsilylhalogenide - and of the conditions, under which the hydrohalogenic acid is generated - for example by adjusting temperature, solvent composition or further parameters as necessary for obtaining a desired salt (e.g. the mono- or dihydrohalide salt) of the organic amine or a desired polymorphic form of the hydrohalide salt of the organic amine. Thus, the present invention relates to the use of a trialkylsilylhalogenide for the preparation of a crystalline hydrohalide of an organic amine. This use is particularly advatageous if the crystalline hydrohalide of the organic amine is one desired hydrohalide among several known hydrohalides of said organic amine, for example in such cases where a crystalline monohydrochloride of a drug is desired, while there is or are also di- or tri-hydrochloride of said drug. Using the conventional technique of adding HCl gas to the crystallization mixture would not be difficult to control with regard to amount of HCl added, would not allow the same flexibility with regard to the choice of crystallization conditions and would simply be more difficult to handle.
A further advantage is that the hydrohalogenic acid can be generated essentially in the absence of water or at least in the presence of only small amounts of water. Thus, anhydrous forms of crystalline hydrohalides of organic amines become accessible. Thus, the invention also relates to the use of a trialkylhalogenide in the preparation of an anhydrous crystalline hydrohalide salt of an organic amine. Furthermore, the generation of controlled amounts of hydrohalogenic acid essentially in the absence of water makes solvates of organic amines accessible which would not form in the presence of water, for example solvates which are thermodynamically less stable than the corresponding hydrates. Thus, the present invention also relates to the use of a trialkylhalogenide in the preparation of solvates of crystalline hydrohalides of an organic amine, in particular the preparation of anhydrous solvates. It will be apparent to the skilled person that preferred trialkaylsilylhalogenides, preferred organic amines and preferred conditions for these uses are as defined above in the description of the process of the invention.

The following examples will illustrate, but do not limit the scope of the present invention. Wherever used, room temperature denotes a temperature in the range of 20 - 30°C.

### EXAMPLES

The infrared spectra were recorded using a BRUKER Tensor 27 FTIR-spectrometer with diamond ATR-cell..
The XRPD were recorded on a AXS-BRUKER X-ray powder diffractometer D-8 using the following acquisition conditions: tube anode: Cu; generator tension: 40 kV; generator current: 40 mA; start angle: 2.0° ?; end angle: 40.0° ?; step size: 0.01° ?; time per step: 2 seconds.
Differential scanning calorimetry (DSC) was performed with a DSC 7 (Perkin-Elmer, Norwalk, Ct., USA) using the Pyris 2.0 software. Samples were weighed into 25 µl Al-Pans. Dry nitrogen is used as the purge gas (purge: 20 ml min⁻¹).
1 H-NMR spectra were recorded on a Brucker AM-300 spectrometer.

### Example 1

### preparation of the Mycophenolate Mofetil hydrochloride in its crystalline anhydrous form

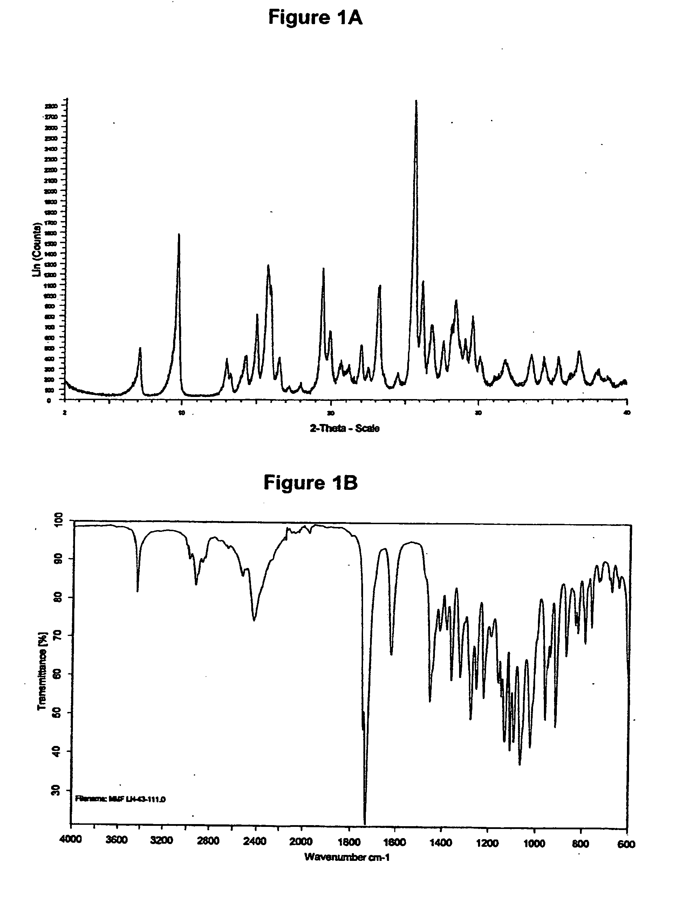

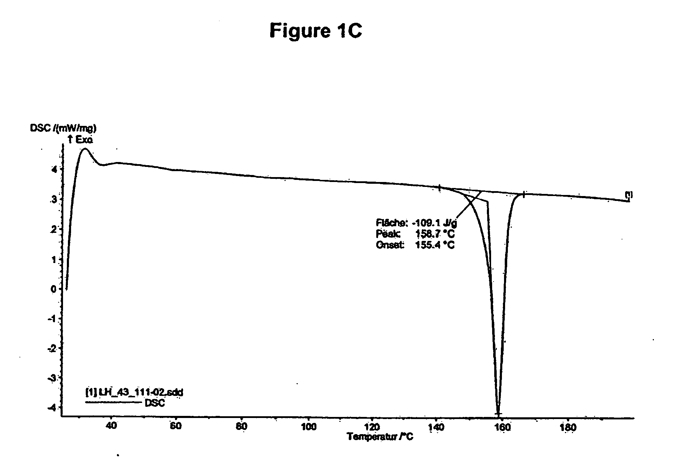

2 g (4,61 mmol) mycophenolate mofetil base were dissolved in 50 ml ethyl acetate at room temperature. To this solution 0,3 ml (1,2 equiv.) acetic acid and 0,7ml (1,2 equiv.) trimethylchlorosilane were added under stirring. After 2 minutes at room temperature the crystallization started. The suspension was stirred for 1 hour and the precipitate filtered off. The solid was washed with ethyl acetate and dried under vacuum at room temperature to yield 2,11g (97,6%) of mycophenolate mofetil hydrochloride.
mp. = 157.2°C
The XRD pattern of mycophenolate mofetil hydrochloride is shown in Figure 1A and corresponds to crystalline anhydrous form with X-ray crystallography data as shown in WO 95/07902. The infrared spectrum obtained is shown in Figure 1B.
DSC of mycophenolate mofetil hydrochloride shows an endotherm peak at about 159°C (onset temperature about 155°C, see Figure 1C).

### Example 2

### preparation of Venlafaxine hydrochloride

### Example 2.a preparation of Venlafaxine hydrochloride form I

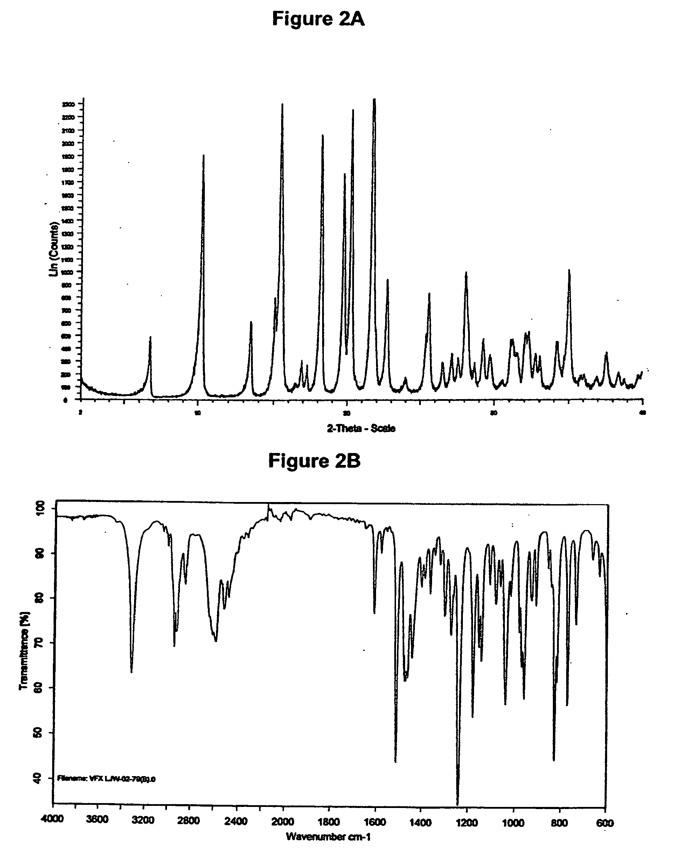

0,4 g (1,44 mmol) Venlafaxine base were dissolved in 10 ml ethyl acetate at room temperature. To this solution 0,1 ml (1,1 equi.) acetic acid and 0,2ml (1,1 equiv.) trimethylchlorosilane were added under stirring. After 2 minutes at room temperature the crystallization started. The suspension was stirred for 30 minutes and the precipitate filtered off. The solid was washed with ethyl acetate and dried under vacuum at room temperature to yield 0,41g (89,1%) of Venlafaxine hydrochloride.
mp. = 208°C
The XRD pattern of Venlafaxine hydrochloride form I is shown in Figure 2A and corresponds to form I with X-ray crystallography data as shown in US 03/0114536.
The infrared spectrum obtained is shown in Figure 2B.

### Example 2.b preparation of Venlafaxine hydrochloride form II

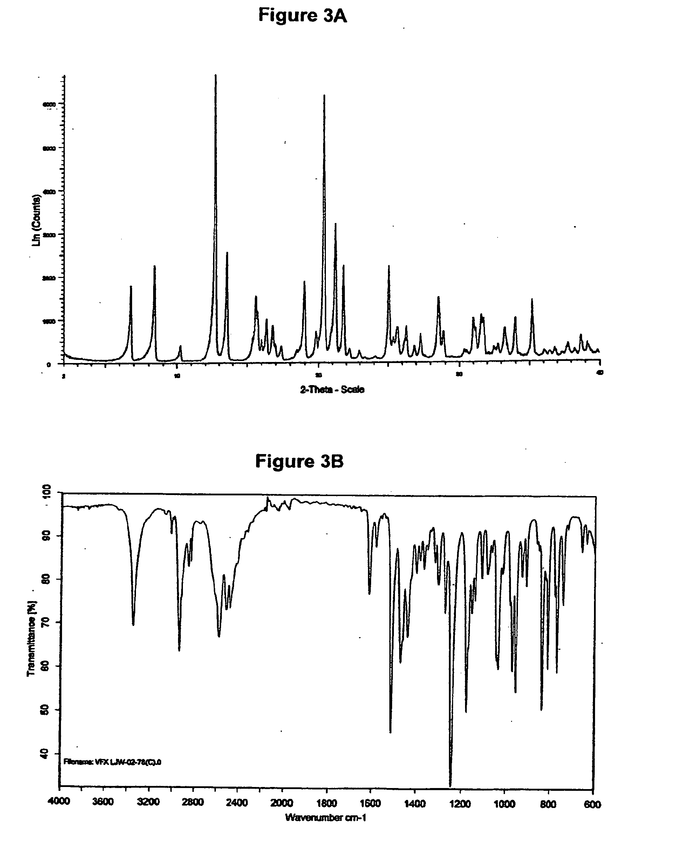

0,4 g (1,44 mmol) Venlafaxine base were dissolved in 10 ml acetone at room temperature. To this solution 0,1 ml (1,1 equiv.) acetic acid and 0,2ml (1,1 equiv.) trimethylchlorosilane were added under stirring. After 2 minutes at room temperature the crystallization started. The suspension was stirred for 30 minutes and the precipitate was filtered off. The solid was washed with ethyl acetate and dried under vacuum at room temperature to yield 0,38g (82,6%) of Venlafaxine hydrochloride form II.
The XRD pattern of Venlafaxine hydrochloride form II is shown in Figure 3A and corresponds to form II with X-ray crystallography data as shown in WO 02/45658.
The infrared spectrum obtained is shown in Figure 3B.

### Example 2.c preparation of Venlafaxine hydrochloride form II

0,4 g (1,44 mmol) Venlafaxine base were dissolved in 10 ml acetonitrile at room temperature. To this solution 0.1 ml (1,1equiv.) acetic acid and 0,2ml (1,1equiv.) trimethylchlorosilane were added under stirring. After 2 minutes at room temperature the crystallization started. The suspension was stirred for 30 minutes and the precipitate was filtered off. The solid was washed with ethyl acetate and dried under vacuum at room temperature to yield 0,23g (51,1 %) of Venlafaxine hydrochloride form II.

### Example 3

### preparation of Sertraline hydrochloride form II using Sertraline base

### Example 3.a

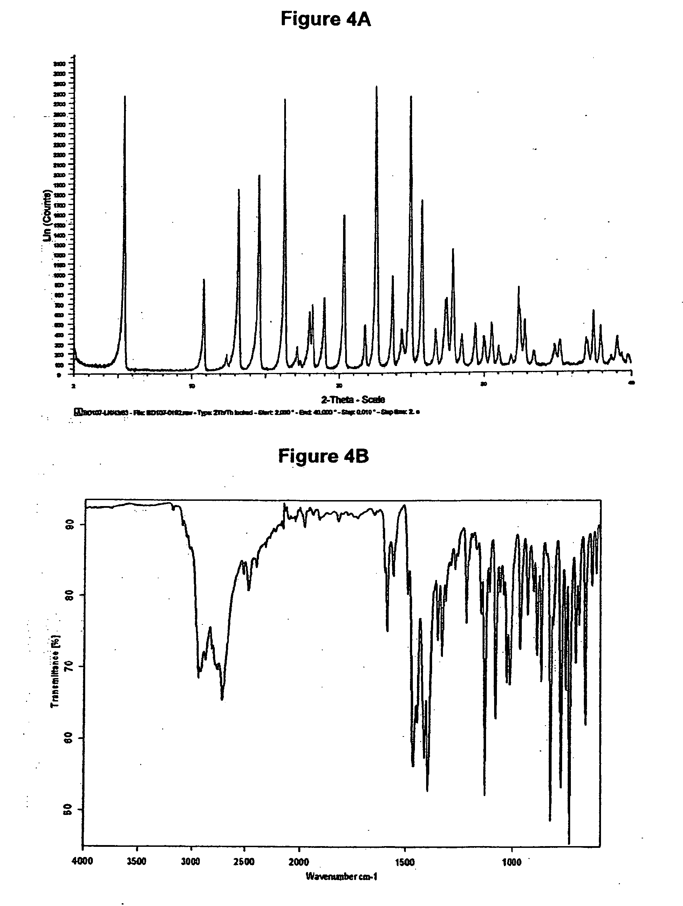

3 g (9,8 mmol) Sertraline base were dissolved in 60 ml acetonitrile at room temperature. To this solution 0,6ml (1eq) acetic acid and 1,4ml (1,1eq) of trimethylchlorosilane was added under stirring. While adding Sertraline hydrochloride precipitated nicely in the crystalline Form II. After stirring the suspension for one hour the product was filtered of and dried at 50°C for 3 hours to yield 3,2g (95,3%) of Sertraline hydrochloride Form II.
mp.: 252°C
The XRD pattern obtained is shown in Figure 4A and corresponds to pure form II.
The infrared spectrum obtained is shown in Figure 4B.

### Example 3.b

3 g (9,8 mmol) Sertraline base were dissolved in a mixture of 60 ml acetonitrile and 1 ml n-butanol. The solution was heated to 50°C and 1,4 ml (1,1eq) trimethylchlorosilane was added under stirring. Immediately during the addition Sertraline hydrochloride precipitated in the crystalline Form II. After stirring for 30 min at 50°C, the suspension was cooled to room temperature and stirred for about one hour. The product was filtered off and dried at 50°C for 3 hours to yield 3,1 g (94%) of Sertraline hydrochloride form II.

### Example 3.c

10 g (32,7 mmol) Sertraline base in 200 ml methyl isobutyl ketone (MIBK) were heated to about 80°C. To the solution 2,4 ml (1.1eq) acetic acid and then 4,5 ml (1,1eq)) trimethylchlorosilane were added under stirring. A gelatinous mass was first obtained which becomes crystalline after stirring at 80°C for one hour. The reaction mixture was cooled to room temperature and again stirred for about one and a half hour. The product was filtered off and dried at 50°C for 4 hours to yield 10,93 g (97,7%) of Sertraline hydrochloride form II.

### Example 4

### preparation of Sertraline hydrochloride form II using Sertraline Mandelate

### Example 4.a

A suspension of 3 g (6,5 mmol) Sertraline Mandelate salt in 60 ml acetonitrile was stirred at room temperature and 1,4 ml (1,7eq) trimethylchlorosilane was added. The viscous suspension changed first to a thin suspension and afterwards a thick suspension of crystals of Sertraline hydrochloride Form II within 15 minutes was obtained. After stirring for about one hour, the product was filtered off and dried at 50°C under vacuum to yield 2,09 g (96,3%) of Sertraline hydrochloride form II.

### Example 4.b

A suspension of 3 g (6,5 mmol) Sertraline Mandelate salt in 60 ml methyl ethyl ketone was heated to about 80°C and 0,9 ml (1,1 eq) trimethylchlorosilane was added. A dear solution was obtained and soon after Sertraline hydrochloride in the crystalline form II starts to precipitate. The reaction mixture was cooled to room temperature under stirring within one hour and then the product was filtered off and dried at 50°C under vacuum to yield 1,97 g (87,8%) of Sertraline hydrochloride form II.

### Example 4.c

A suspension of 3 g (6,5 mmol) Sertraline Mandelate salt in 60 ml methyl isobutyl ketone was heated to about 80°C and 0,9 ml (1,1 eq) trimethylchlorosilane is added. The suspension changed to a gelateneous mass. After approximately 5 minutes Sertraline hydrochloride in the crystalline form II started to form. The reaction mixture was stirred at 80°C for about 20 minutes and then cooled to room temperature under stirring within one hour. The product was filtered off and dried at 50°C under vacuum for 3 hours to yield 2,11 g (94,0%) of Sertraline hydrochloride form II.

### Example 4.d

A suspension of 15 g (32,7 mmol) Sertraline Mandelate salt in 300 ml methyl isobutyl ketone was heated to about 100°C and 4,6 ml (1,1 eq) trimethylchlorosilane was added. The suspension changed to a gelateneous mass. After approximately 5 minutes Sertraline hydrochloride in the crystalline form II started to form The suspension changed to a gelateneous mass. After approximately 5 minutes. The reaction mixture was stirred at 100°C for about 15 minutes and then cooled to room temperature under stirring within one and a half hour. The product was filtered off, washed twice with 10 ml acetone and dried at 50°C under vacuum for 4 hours to yield 10,64 g (94,9%) of Sertraline hydrochloride form II.

### Example 5

### preparation of Sertraline hydrochloride form II using Sertraline Oxalate

### Example 5.a

### preparation of Sertraline Oxalate

To a solution of 3 g (9,8 mmol) Sertraline free base in 50 ml ethyl acetate was added a solution of 0,97 g (1,1 eq) oxalic acid in 50 ml methanol. After stirring for 15 minutes the crystalline precipitate was filtered off and dried at 50°C under vacuum to yield 3,37 g (86,8%) of Sertraline Oxalate.

### Example 5.b

### preparation of Sertraline hydrochloride form II from Sertraline oxalate

A suspension of 0,7 g (1,77 mmol) Sertraline Oxalate in 15 ml acetonitrile was stirred at room temperature and 250 µl (1,1 eq) trimethylchlorosilane was added. After addition the suspension converted almost to a solution but at the same time Sertraline hydrochloride in the crystalline form **II** started to precipitate. After stirring for about one hour, the product was filtered off and dried at 50°C under vacuum to yield 0,47 g (77,8%) of Sertraline hydrochloride form II.

### Example 6

### preparation of Sertraline hydrochloride form I.

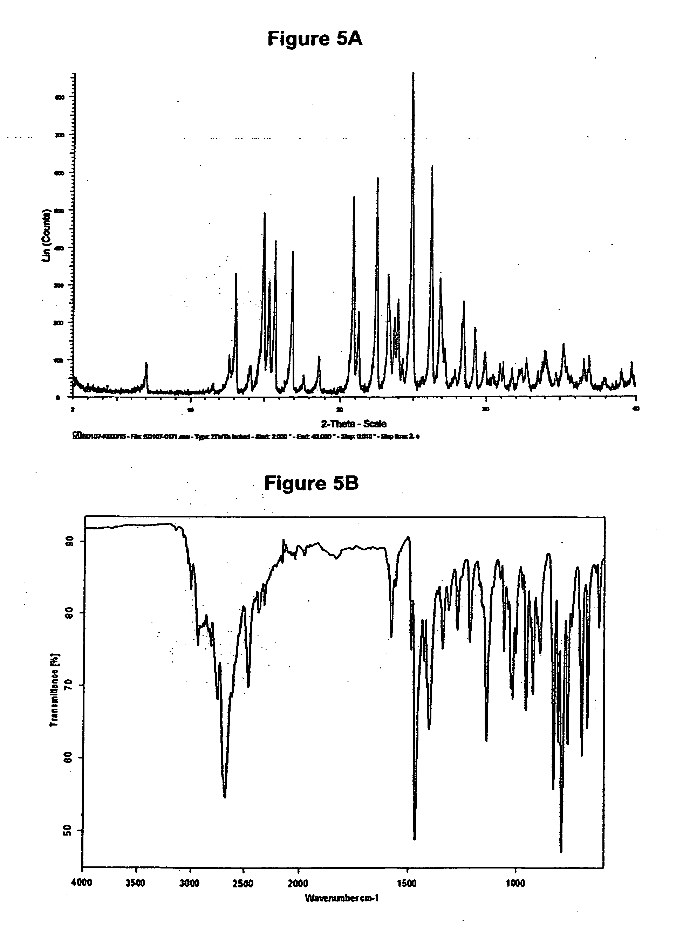

A solution of 3 g (9,8 mmol) Sertraline base in 60 ml 2-propanol was stirred at room temperature and 0,6 ml acetic acid and 1,4 ml (1,1 eq) trimethylchlorosilane were added under stirring. After 2 minutes at room temperature the crystallization started. The suspension was stirred for 60 minutes and the precipitate filtered off. The solid was washed with 2-propanol and dried under vacuum at 50°C for 3 hours to yield 2,95g (87,8%) of Sertraline hydrochloride form I.
The XRD pattern obtained is shown in Figure 5A and corresponds to form I.
The infrared spectrum obtained is shown in Figure 5B.

### Example 7

### preparation of Donepezil hydrochloride

### Example 7.a

### preparation of Donepezil hydrochloride form II

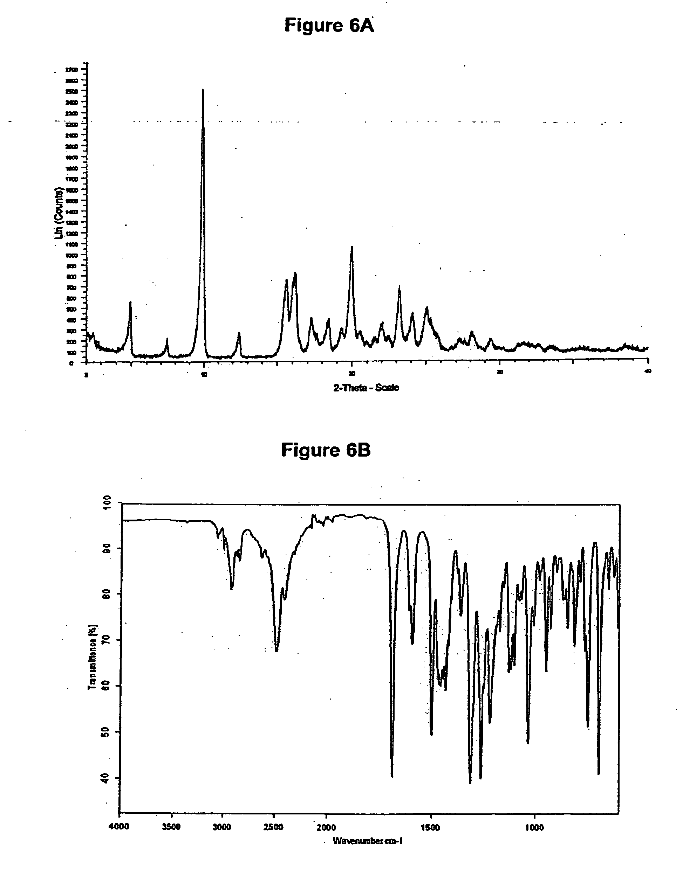

0,5 g (1,32 mmol) Donepezil base were dissolved in 30 ml ethyl acetate at room temperature. To this solution 0,1ml (1,1 eq) acetic acid and 0,2ml (1,1 eq) trimethylchlorosilane were added under stirring. After 2 minutes at room temperature the crystallization started. The suspension was stirred for 2 hours and the precipitate was filtered off. The solid was washed with ethyl acetate and dried under vacuum at room temperature to yield 0,55g (100%) of Donepezil hydrochloride form II.
The XRD pattern obtained is shown in Figure 6A and corresponds to form II.
The infrared spectrum obtained is shown in Figure 6B.

### Example 7.b

### preparation of Donepezil hydrochloride form III

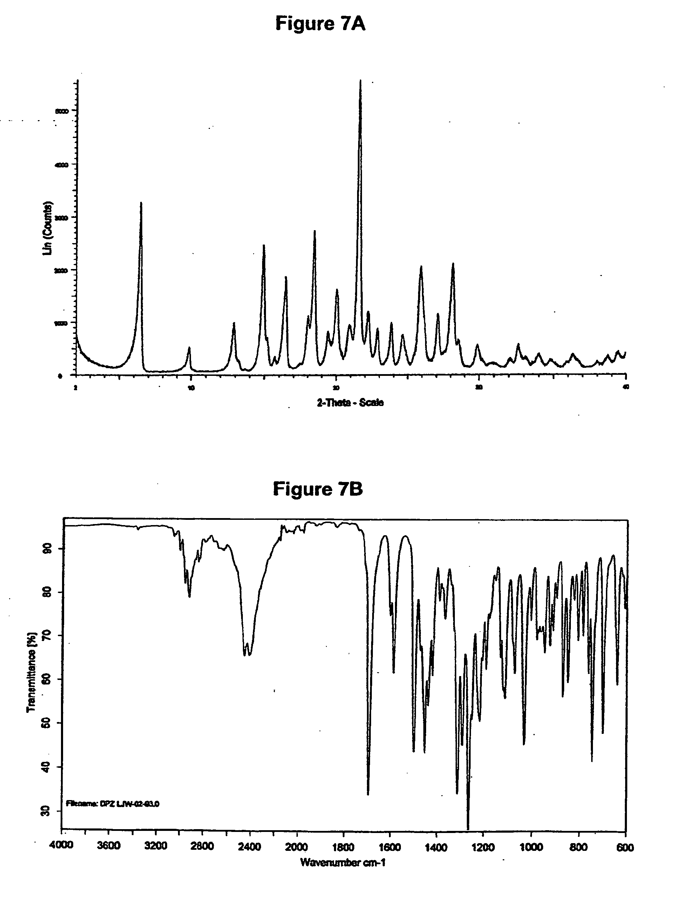

0,5 g (1,32 mmol) Donepezil base were dissolved in 10 ml acetone at room temperature. To this solution 0,1ml (1,1 eq) acetic acid and 0,2ml (1,1 eq) trimethylchlorosilane were added under stirring. After 2 minutes at room temperature the crystallization started. The suspension was stirred for 30 minutes and the precipitate filtered off. The solid was washed with ethyl acetate and dried under vacuum at room temperature to yield 0,54g (98,5%) of Donepezil hydrochloride.
mp.=211°C
The XRD pattern obtained is shown in Figure 7A and corresponds to form III.
The infrared spectrum obtained is shown in Figure 7B.

### Example 7.c

### preparation of Donepezil hydrochloride form III

Example 7.b was repeated by using aetonitrile instead of acetone.
Yield: 0,47g (85,6%) of donepezil hydrochloride form III.

### Example 8

### preparation of Terbinafine hydrochloride

### Example 8.a

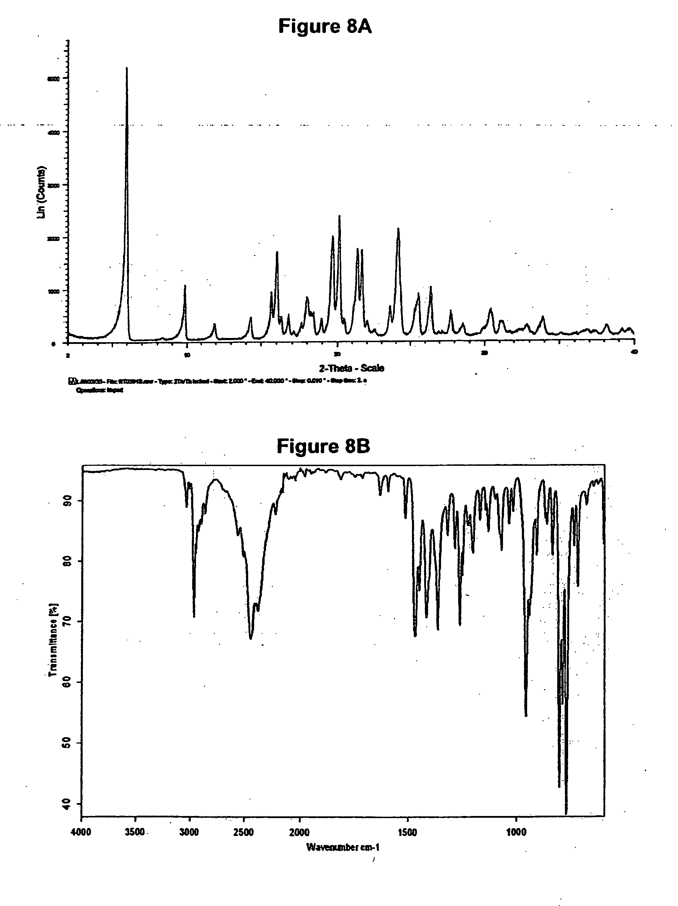

0,4 g (1,37 mmol) Terbinafine base were dissolved in 5 ml acetone at room temperature. To this solution 86 µl (1.1 eq) acetic acid and 191 µl (1,1 eq) trimethylchlorosilane were added under stirring. After 2 minutes at room temperature the crystallization started. The suspension was stirred for 1h and the precipitate was filtered off. The solid was washed with acetone and dried under vacuum at room temperature to yield 0,3g (66,7%) of Terbinafine hydrochloride.
mp.= 185°C
The XRD pattern of Terbinafine hydrochloride is shown in Figure 8A and corresponds to literature data (Cryst. Eng. Comm., 2002, 4(67), 393-400). The infrared spectrum obtained is shown in Figure 8B.

### Example 8.b

Example 8.a was repeated by using acetonitrile instead of acetone.
Yield: 0,19g (42,2%) of Terbinafine hydrochloride

### Example 8.c

Example 8.a was repeated by using tert.-butyl-methyl-ether instead of acetone.
Yield: 0,41g (91,1%) of Terbinafine hydrochloride

### Example 9

### preparation of Cinacalcet hydrochloride

### Example 9.a

1,0 g (2,80 mmol) Cinacalcet base were dissolved in 10 ml acetonitile at room temperature. To this solution 0,19 ml (1,2 eq) acetic acid and 0.42 ml (1,2 eq) trimethylchlorosilane were added under stirring. After 2 minutes at room temperature the crystallization started. The suspension was stirred for 2h and the precipitate was filtered off. The solid was washed with acetonitrile and dried under vacuum at room temperature to yield 0,45g (40,8%) of Cinacalcet hydrochloride.
mp. = 173°C
The XRD pattern obtained is shown in Figure 9A and the infrared spectrum obtained is shown in Figure 9B.

### Example 9.b

Example 9.a was repeated by using ethylacetate instead of acetonitrile.
Yield: 0,41g (37,2%) of Cinacalcet hydrochloride.

### Example 10

### preparation of Citalopram hydrobromide

### Example 10.a

0,72 g (22,2 mmol) Citalopram base were dissolved in 10 ml acetonitrile at room temperature. To this solution 0,14 ml (1,1 eq) acetic acid and 0,32 ml (1,1 eq) trimethylbromosilane were added under stirring. After 30 minutes at room temperature 15 ml of diethylether were added and the crystallization started. The suspension was stirred for 1 h and the precipitate filtered off. The solid was washed with acetonitrile and dried under vacuum at room temperature to yield 0,67g (74.5%) of Citalopram hydrobromide.
mp.= 182°C
The XRD pattern obtained is shown in Figure 10A and the infrared spectrum obtained is shown in Figure 10B.

### Example 10.b

Example 10.a was repeated with 0,5 g (15,4 mmol) Citalopram base using ethylacetate instead of acetonitrile as solvent.
Yield: 0,53g (84,8%) of Citalopram hydrobromide.

### Example 10.c

Example 10.a was repeated with 0,59 g (18,2 mmol) Citalopram base using acetone instead of acetonitrile as solvent.
Yield: 0,67g (90,9%) of Citalopram hydrobromide.

### Example 10.d

0,27 g (0,8 mmol) Citalopram base were dissolved in 3 ml isopropanol at room temperature and 145 µl (1,1 eq) trimethylbromosilane were added to the solution. After standing in a refrigerator over night the crystalline precipitate was filtered off and dried in vacuum to yield 0,25g (74,1 %) of Citalopram hydrobromide.

### Example 11

### preparation of Aripiprazole hydrochloride Form A

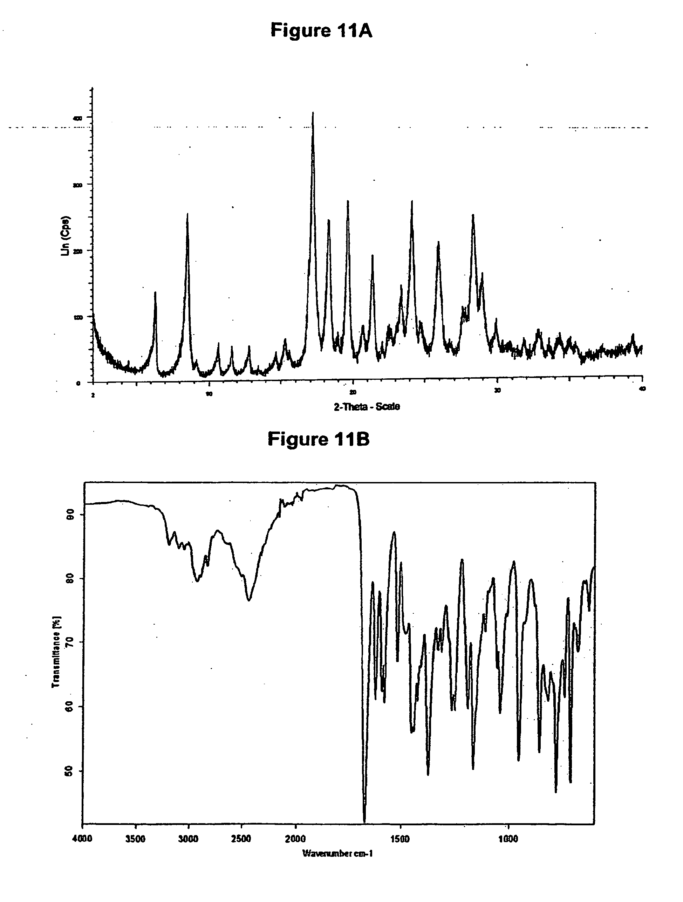

2,0 g (4,46 mmol) aripiprazole were dissolved in 20 ml 1,2-dichloromethane at room temperature. To this solution 0,45ml (1,1 eq) n-butanol and 0,63 ml (1,1 eq) trimethylchlorosilane were added under stirring. After 2 minutes at room temperatur the crystallization started. The suspension was stirred for 15 minutes and the precipitate was filtered off. The solid was washed with 1,2-dichloromethane and dried under vacuum at room temperature to yield 2,05g (94.0%) of aripiprazole hydrochloride.
mp.= 210°C
The XRD pattern of the product is shown in Figure 11A and corresponds to XRPD data of Aripiprazole hydrochloride Form A as shown in WO 2004/083183 (Hetero Drugs Ltd.). The infrared spectrum obtained is shown in Figure 11B.

### Example 12

### preparation of Pramipexole Monohydrochloride

0,5 g (2,37 mmol) Pramipexole base were dissolved in 20 ml acetonitrile at room temperature. To this solution 0,24 ml n-butanol (2,6 mmol, 1,1 equiv.) and 0,33 ml trimethylchlorosilane (2,6 mmol, 1,1 equiv.) were added under stirring. After 1 minute at room temperatur the crystallization started. The suspension was stirred for 1 hour and the precipitate was filtered off. The solid was washed with acetonitrile and dried under vacuum at room temperature to yield 0,56g (95,5%) of Pramipexole Monohydrochloride.
mp.= 264°C
The XRD pattern obtained is shown in Figure 12A and the infrared spectrum obtained is shown in Figure 12B.

### Example 13

### preparation of Moxifloxacine hydrochloride anhydrous form IV

### Example 13.a

### Moxifloxacine hydrochloride methylene dichloride solvate

5 g (12,5 mmol) Moxifloxacin were prepared according to EP 550903 and dissolved in 50 ml methylene dichloride at room temperature. To this solution 0,9 ml (1,2 equiv.) acetic acid and 2 ml (1,2 equiv.) trimethylchlorosilane were added under stirring. Immediately after addition of the chlorosilane the crystallization started. The suspension was stirred for about 30 min and the precipitate filtered off and dried under vacuum at room temperature to yield 6,3g (94,2%) of the 1:1 solvate of Moxifloxacine hydrochloride with methylene dichloride.
¹H-NMR (DMSO-d₆): 0.8 - 0.95 (m, 1H), 0.95 -1.3 (m. 3H), 1.6 -1.9 (m, 4 H), 2.55-2.75 (m, 1H), 2.8-3.0(m, 1H), 3.5 - 3.7 (m with Singlet at 3.6 ppm, 4H), 3.7 - 3.8 (m, 1H), 3.8-4.0 (m, 2H), 4.0-4.2 (m, 2H), 5.77 (s, 2H), 7.65 (d, J = 14 Hz, 1H), 8.66 (s, 1H), 8.95 (s, broad, 1H), 10.2 (s, broad, 1H), 15.1 (s, broad, 1H).
The singlet at 5.77 ppm corresponds to about one mol methylene dichloride per mol of substance (see Figure 13C).
The XRD pattern of the product is shown in Figure 13A and the infrared spectrum obtained is shown in Figure 13B.
The Moxifloxacine hydrochloride solvate with methylene dichloride is not hygroscopic (no water uptake after 1 day at 33% relative humidity.

### Example 13.b

### Moxifloxacine hydrochloride anhydrous form IV

2 g Moxifloxacine hydrochloride methylene dichloride solvate were dried in vacuum at 100°C for about 6 hours yielding 1,75 g of the desolvated anhydrous form IV of Moxifloxacine hydrochloride.
The XRD pattern of the product is shown in Figure 14A and the infrared spectrum obtained is shown in Figure 14B.

### Example 14

### preparation of Moxifloxacine hydrochloride solvate with acetic acid

### Example 14.a

3,0 g (7,47 mmol) moxifloxacin were prepared according to EP 550903 and dissolved in 30 ml acetic acid at room temperature. To this solution 2,0 ml (2,1 eq) trimethylchlorosilane were added under stirring. 30 minutes after addition of the chlorosilane the crystallization started. The suspension was stirred for about 3,5 hours and the precipitate filtered off washed with acetonitrile and dried under vacuum at room temperature to yield 3,21g (86,3%) of the 1:1 solvate of Moxifloxacine hydrochloride with acetic acid.
¹H-NMR (DMSO-d6): 0.8 - 0.95 (m, 1H), 0.95 -1.3 (m, 3H), 1.6 -1.9 (m, 4 H), 1.9 (s, 3H), 2.55 - 2.75 (m, 1H), 2.8-3.0 (m, 1H), 3.1- 3.25 (m, 1H), 3.5 - 3.7 (m with Singlet at 3.6 ppm, 4H), 3.7 - 3.8 (m, 1H), 3.8 - 4.0 (m, 2H), 4.0 - 4.2 (m, 2H), 7.63 (d, J =14 Hz, 1H), 8.65 (s, 1H), 9.1 (s, broad, 1H), 10.4 (s, broad, 1H), 12.7 (s, broad, 1H).
The singlet at 1.9 ppm corresponds to about one mol acetic acid per mol of substance (see Figure 15C).
The XRD pattern of the product is shown in Figure 15A and the infrared spectrum obtained is shown in Figure 15B.

### Example 14.b

1,0 g (2,49 mmol) Moxifloxacin were prepared according to EP 550903 and dissolved in 5 ml acetic acid and 5 ml acetonitrile at room temperature. To this solution 0,38 ml (1,2 eq) trimethylchlorosilane were added under stirring.
2 hours after addition of the chlorosilane the crystallization didn't start. To this solution 0,38 ml trimethylchlorosilane were once more added under stirring. The suspension was stored for about 17 hours at 4°C and the precipitate filtered off washed with acetonitrile and dried under vacuum at room temperature to yield 0,93g (75,0%) of the 1:1 solvate of Moxifloxacine hydrochloride with acetic acid.

### Example 15

### preparation of Moxifloxacine hydrochloride solvate with nitromethane

0,5 g (1,25 mmol) Moxifloxacin were prepared according to EP 550903 and dissolved in 15 ml nitromethane at appr. 60°C. To this solution 0,14 ml (2 eq) acetic acid and (2 eq) ml trimethylchlorosilane were added under stirring. The suspension was stored for about 17 hours and the precipitate was filtered off, washed with acetone and dried under vacuum at room temperature to yield 0,55g (88,2%) of the 1:1 solvate of Moxifloxacine hydrochloride with nitromethane.
¹H-NMR (DMSO-d6): 0.8 - 0.95 (m, 1H), 0.95 -1.3 (m, 3H), 1.6 -1.9 (m, 4 H), 2.55 - 2.75 (m, 1H), 2.8 - 3.0 (m, 1H), 3.1 - 3.25 (m,1H). 3.5 - 3.7 (m with Singlet at 3.6 ppm, 4H), 3.7 - 3.8 (m, 1H), 3.8 - 3.95 (m, 2H), 4.0 - 4.2 (m, 2H), 4.44 (s, 3H), 7.63 (d, J =14 Hz, 1H), 8.65 (s, 1H), 9.0 (s, broad, 1H), 10.3 (s, broad, 1H), 15.1 (s, broad, 1H).
The singlet at 4.44 ppm corresponds to about one mol nitromethane per mol of substance (see Figure 16C).
The XRD pattern of the product is shown in Figure 16A and the infrared spectrum obtained is shown in Figure 16B.

### Example 16

### preparation of Duloxetine hydrochloride

### Example 16.a

0,3 g (1,0 mmol) Duloxetine base were dissolved in 5 ml ethylacetate at room temperature. To this solution 65 µl acetic acid and 0,14 ml trimethylchlorosilane were added under stirring. After addition of the chlorosilane a precipitate was formed and the suspension was stirred for about 2 hours at room temperature. The white crystalline solid was filtered off and dried under vacuum at room temperature to yield 0,21g (62.9%) of Duloxetine hydrochloride
mp.= 161°C
The XRD pattern of the product is shown in Figure 17A and the infrared spectrum obtained is shown in Figure 17B.

### Example 16.b

Example 16.a was repeated by using acetone instead of ethylacetate.
Yield: 0,26g (77,9%) of Duloxedine hydrochloride.

### Example 17

### preparation of Linezolid hydrochloride

### Example 17.a

5 g (14,8 mmol) Linezofide were dissolved in 200 ml acetonitrile at room temperature. To this solution 1,0 ml (1,1equiv.) acetic acid and 2,1 ml (1,1equiv.) trimethylchlorosilane were added under stirring. After addition of the chlorosilane and stirring for 1 hour a precipitate was formed and the suspension was stirred for about 3,5 hours at room temperature. The white crystalline solid was filtered off and dried under vacuum at room temperature to yield 1,43g (25,8%) of linezolide hydrochloride.
mp.= 163°C
The XRD pattern of the product is shown in Figure 18A and the infrared spectrum obtained is shown in Figure 18B.

### Example 17.b

Example 17.a was repeated with 0,5 g (1,5 mmol) Linezolid using acetone instead of acetonitrile as solvent.
Yield: 0,20g (36.4%) of Linezolide hydrochloride.

### Example 18

### preparation of Memantine hydrochloride

0,5 g (2,8 mmol) Memantine base were dissolved in 10 ml ethylacetate at room temperature. To this solution 0,1 ml (1,1 equiv.) methanol and 0,4 ml (1,1 equiv.) trimethylchlorosilane were added under stirring. After addition of the chlorosilane a precipitate was formed and the suspension was stirred for about 2 hours at room temperature. The white crystalline solid was filtered off and dried under vacuum at room temperature to yield 0,59g (98,0%) of the Memantine hydrochloride.
mp.= 293-296°C
The XRD pattern of the product is shown in Figure 19A and the infrared spectrum obtained is shown in Figure 19B.

### Example 19

### Preparation of Rimonabant hydrochloride form I

### Example 19a

1 g (2,16 mmol) Rimonabant was suspended in 20 ml acetonitrile at room temperature. To the suspension 0,105 ml (1,2 equiv.) methanol and 0,33 ml (1,2 equiv.) trimethylchlorosilane were added under stirring. A clear solution was obtained and soon after Rimonabant hydrochloride in the crystalline form I started to precipitate. The product was filtered off and dried at room temperature under vacuum over night to yield 0,9 g (83,4%) of Rimonabant hydrochloride form I.
The XRD pattern of the product is shown in Figure 20A and the infrared spectrum obtained is shown in Figure 20B.

### Example 19b

1 g (2,16 mmol) Rimonabant was dissolved in 10 ml ethyl acetate at room temperature To this solution 0,105 ml (1,2 equiv.) methanol and 0,33 ml (1,2 equiv.) trimethylchlorosilane were added under stirring. After addition of the chlorosilane a precipitate was formed and the white crystalline solid was filtered off and dried under vacuum at room temperature to yield 0,95g (88,1%) of Rimonabant hydrochloride form I.

### Example 19c

Example 19b was repeated using acetone instead of ethyl acetate as solvent.
Yield: 0,89g (82,5%) of Rimonabant hydrochloride form I.

### Example 20

### Preparation of Clopidogrel hydrochloride form I

### Example 20a

1,2 g (3,73 mmol) Clopidogrel were dissolved in 10 ml acetone at room temperature. To the solution 255 µl (1,2 equiv.) acetic acid and 565 µl (1,2 equiv.) trimethylchlorosilane
were added under stirring. After addition of 6 ml diisopropylether Clopidogrel hydrochloride started to precipitate. After stirring for one hour the crystalline precipitate was filtered off and dried at room temperature under vacuum over night to yield 0,85 g (63,8%) Clopidogrel hydrochloride form I.
The XRD pattern of the product is shown in Figure 21A and the infrared spectrum obtained is shown in Figure 21B

### Example 20b

1 g (3,11 mmol) Clopidogrel was dissolved in 10 ml acetonitrile at room temperature. To the solution 213 µl (1,2 equiv.) acetic acid and 475 µl (1,2 equiv.) trimethylchlorosilane
were added under stirring. The mixture was cooled down to about 5°C and 40 ml diisopropylether was added under stirring. After stirring for one hour the crystalline precipitate was filtered off and dried at room temperature under vacuum over night to yield 0,50 g (45.0%) Clopidogrel hydrochloride form I.

### Example 20c

1 g (3,11 mmol) Clopidogrel was dissolved in 10 ml ethyl acetate at room temperature. To the solution 213 µl (1,2 equiv.) acetic acid and 475 µl (1,2 equiv.) trimethylchlorosilane
were added under stirring. After addition of the chlorosilane a viscous solid precipitated which converted to a crystalline product within about 5 min. The mixture was stirred at room temperature for one hour. The product was then filtered off and dried at room temperature under vacuum over night to yield 0,88 g (79,3%) Clopidogrel hydrochloride form I.

### Example 20d

30 g (93,22 mmol) Clopidogrel free base were dissolved in 1000 ml toluene in a 1 Liter reaction flask equipped with a mechanical stirrer and a dropping funnel. To the solution was added 4,5 ml (1,2 equiv.) methanol and after mixing by stirring 14,1 ml (1,2 equiv.) trimethylchlorosilane was added dropwise at room temperature under slowly stirring within 45 min. After addition the reaction mixture was stirred at room temperature for about three hours wherein the fluffy precipitate converted to a white crystalline solid. The salt was filtered off and washed with 20 ml toluene The product was dried under vacuum at room temperature over night yielding 29,1 g (87,1 %) Clopidogrel hydrochloride form I.

### Example 21

### Preparation of Clopidogrel hydrobromide form A

### Example 21a

1 g (3,11 mmol) Clopidogrel was dissolved in 10 ml ethyl acetate at room temperature. To the solution 213 µl (1,2 equiv.) acetic acid and 482 µl (1,2 equiv.) trimethylbromosilane
were added under stirring. After addition of the bromosilane a viscous solid precipitated which converted to a crystalline product within about 30 min. The mixture was stirred at room temperature for one hour. The product was then filtered off and dried at room temperature under vacuum over night to yield 1,03 g (82.3%) Clopidogrel hydrobromide form A.
The XRD pattern of the product is shown in Figure 22A and the infrared spectrum obtained is shown in Figure 22B

### Example 21 b

1 g (3,11 mmol) Clopidogrel was dissolved in 10 ml isopropanol. To the solution 482 µl (1,2 equiv.) trimethylbromosilane were added. After stirring for 90 min the product crystallized after scratching with a glass stick. The hydrobromide salt was filtered off and dried at room temperature under vacuum over night to yield 0,86 g (68,7%) Clopidogrel hydrobromide form A.

### Example 22

### Preparation of Prasugrel Hydrochloride Form B

5,0 g (13,4 mmol) Prasugrel base were dissolved in 75 ml acetone at room temperature. To the solution 919 µl (1,2 equiv.) acetic acid and 2053 µl (1,2 equiv.) trimethylchlorosilane
were added under stirring. Immediately after addition a white precipitate was obtained. The hydrochloride salt wais filtered off and dried at room temperature under vacuum for 6 hours to yield 4,41 g (80,4%) Prasugrel hydrochloride form B. The XRD pattern of the product is shown in Figure 23A and the infrared spectrum obtained is shown in Figure 23B

### Example 23

### Preparation of Prasugrel Hydrochloride acetonitrile solvate

0,5 g (1,34 mmol) Prasugrel base were dissolved in 7,5 ml acetonitrile. The unsolved part was filtered off. To the solution 82 µl (1,2 equiv.) acetic acid and 205 µl (1,2 equiv.) trimethylchlorosilane were added under stirring. The mixture was stirred at room temperature for seventeen hours. The suspension was filtered off and dried at room temperature under vacuum over night to yield 0,43 g (78%) Prasugrel Hydrochloride acetonitrile solvate.
¹H-NMR (DMSO-d₆): 0.84 - 0.97 (m, 2H), 0.98 -1.16 (m, 2H),1.87 - 2.0 (m, 1H), 2.08 (s, 2H), 2.29 (s, 3 H), 2.96 - 3.15 (m, 2H), 3.34 - 3.6 (m, 1H), 3.7-4.2 (m, 3H), 6.14 (s, broad, 1H), 6.58 (s, 1 H), 7.3 - 7.55 (m, 2H), 7.55 - 7.8 (m, 2H).
The singlet at 2.08 ppm corresponds to about one mol acetonitrile per one and a half mol of substance. The XRD pattern of the product is shown in Figure 24A and the infrared spectrum is shown in Figure 24B

### Example 24

### Preparation of Raloxifene Hydrochloride form A

### Example 24a

1,0 g (1,77 mmol) Raloxifene lactate was suspended in 10 ml acetonitrile at room temperature. To the suspension 272 µl (1,2 equiv.) trimethylchlorosilane were added under stirring. After stirring for one hour the crystalline precipitate was filtered off and dried at room temperature under vacuum over night to yield 0,80 g (82,5%) Raloxifene Hydrochloride form A.
The XRD pattern of the product is shown in Figure 25A and the infrared spectrum obtained is shown in Figure 25B

### Example 24b

1,5 g (2,66 mmol) Raloxifene lactate were suspended in 30 ml methyl isobutyl ketone (MIBK) at room temperature. The suspension was heated to 100°C. To the suspension 408 µl (1,2 equiv.) trimethylchlorositane were added under stirring. The suspension was cooled down to room temperature. After stirring for one hour the crystalline precipitate was filtered off and dried at room temperature under vacuum over night to yield 1,42 g (97,6%) Raloxitene Hydrochloride form A.

### Example 24c

0,51 g (0,90 mmol) Raloxifene base were suspended in 5 ml ethanole at room temperature. To the suspension 162 µl (1,2 equiv.) trimethylchlorosilane were added under stirring. A dear solution was obtained and soon after Raloxifene hydrochloride in the crystalline form started to precipitate. After stirring for three hours the crystalline precipitate was filtered off and dried at room temperature under vacuum over night to yield 0,41 g (74,6%) Raloxifene Hydrochloride form A.

### Example 25

### Preparation of Raloxifene hydrochloride THF hemisolvate

### Example 25a

0,50 g (0,88 mmol) Raloxifene base were suspended in 5 ml tetrahydrofuran at room temperature. To the suspension 51 µl (1,2 equiv.) methanol and 162 µl (1,2 equiv.) trimethylchlorosilane were added under stirring. After stirring for three hours at room temperature the crystalline precipitate was filtered off and dried at room temperature under vacuum over night to yield 0.45 g (97,6%) Raloxifene Hydrochloride THF hemisolvate.
¹H-NMR (DMSO-d₆): 1.25 -1.4 (m, 1H), 1.6 -1.9 (m, 7H), 2.8 - 3.1 (m, 2H), 3.3 - 3.55 (m, 4H), 3.55 - 3.65 (m, 2.6 H), 4.4 (t, broad, 2H), 6.7 - 6.8 (m, 2H), 6.9 (dd, J = 2.26 and 8.8 Hz, 1 H), 6.98 (d, J = 9 Hz, 1H), 7.15 - 7.22 (m, 2H), 7.3 (d, J = 8.7 Hz, 1H), 7.4 (d, J = 2.3 Hz, 1H), 7.71 (d, J = 9 Hz, 1H), 9.95 (d, J = 9 Hz, 1H), 10.8 (s. braod, 1H).
The amount of tetrahydrofuran present in the crystalline material wais about 0,6 molar equivalents as determined by proton NMR spectroscopy.
The XRD pattern of the product is shown in Figure 26A and the infrared spectrum obtained is shown in Figure 26B.

### Example 25b

0,30g (0,53 mmol) Raloxifene lactate were suspended in 3 ml tetrahydrofuran at room temperature. To the suspension 82 µl (1,2 equiv.) trimethylchlorosilane were added under stirring. After stirring for two hours at room temperature the crystalline precipitate was filtered off and dried at room temperature under vacuum over night to yield 0,28 g (102,6%) Raloxifene Hydrochloride THF hemisolvate.

### Example 26

### Preparation of Olanzapine Dihydrochloride

### Example 26a

1,0 g (3,20 mmol) Olanzapine base were suspended in 10 ml acetonitrile at room temperature. To the suspension 312 µl (2,4 equiv.) methanol 980 µl (2,4 equiv.) trimethylchlorosilane were added under stirring. After stirring for one hour the crystalline precipitate was filtered off and dried at room temperature under vacuum over night to yield 1,24 g (100,54%) Olanzapine Dihydrochloride form 1. The XRD pattern of the product is shown in Figure 27A and the infrared spectrum obtained is shown in Figure 27B

### Example 26b

1,0 g (3,20 mmol) Olanzapine base were suspended in 10 ml acetone at room temperature. To the suspension 312 µl (2,4 equiv.) methanol 980 µl (2,4 equiv.) trimethylchlorosilane were added under stirring. After stirring for one hour the crystalline precipitate was filtered off and dried at room temperature under vacuum over night to yield 1,35 g (107,31%) Olanzapine dihydrochloride form 1.

### Example 27

### Preparation of Darifenacin Hydrobromide

0,9 g (2,11 mmol) Darifenacin base were dissolved in 9 ml methyl ethyl ketone at room temperature. To the solution 103 µl (1,2 equiv.) methanol 329 µl (1,2 equiv.) trimethylbromosilane were added under stirring. After stirring for one hour the crystalline precipitate was filtered off and dried at room temperature under vacuum over night to yield 0,88 mg (82,13%) Darifenacin Hydrobromide.
The XRD pattern of the product is shown in Figure 28A and the infrared spectrum obtained is shown in Figure 28B

### Example 28

### Preparation of anhydrous Sitagliptin Hydrochloride in amorphous form

0,085 g (0,21 mmol) Sitagliptin base were dissolved in 2 ml Diethylether and 3 ml Methylenchloride at room temperature. To the solution 10 µl (1,2 equiv.) methanole and 32 µl (1,2 equiv.) trimethylchlorosilane were added under stirring. Immediately after addition a precipitate was obtained. The suspension was stirred for 1 hour, filtered off and dried at room temperature under vacuum over night to yield 0,089 g (96,8%) Sitagliptin Hydrochloride in amorphous form.
The XRD pattern of the product is shown in Figure 29A and the infrared spectrum obtained is shown in Figure 29B

### Example 29

### Preparation of anhydrous Vardenafil Dihydrochloride

0,2g (0,359 mmol) Vardenafil, base were suspended in 4 ml diethylether at room temperature. To the suspension 5 ml methylene dichloride were added and a solution was obtained.
35µl (2,4 equiv.) methanol and 110 µl (2,4 equiv.) trimethylchlorosilane were added to the solution and immediately after addition a white precipitate was obtained. The suspension was stirred for twenty minutes, filtered off and dried at room temperature under vacuum over night to yield 0,2g (95,4%) Vardenafil Dihydrochloride.
The XRD pattern of the product is shown in Figure 30A and the infrared spectrum obtained is shown in Figure 30B

### Example 30

### Preparation of Erlotinib Hydrochloride Form A

0,3g (0,82 mmol) Erlotinob base were suspended in 3 ml isopropanol. 126µl (1,2eq.) trimethylchlorosilane are added to the suspension. A white precipitate was obtained. After stirring for 1 hour, the precipitate was filtered off and dried at room temperature under vacuum over night to yield 0,26g (80,8%) Erlotinib Hydrochloride Form A.
The XRD pattern of the product is shown in Figure 31A and the infrared spectrum obtained is shown in Figure 31B

## Claims

1. Process for the preparation of a crystalline hydrohalide of an organic amine wherein a trialkylsilylhalogenide is added to the organic amine in a solvent, which organic amine is in the form of the free base or an acid addition salt, wherein, when the organic amine is in the form of an acid addition salt, the conjugated acid of the acid addition salt is weaker than the hydrohalogenic acid.

2. The process according to claim 1, which process comprises the steps of:
(a) dissolving or suspending the organic amine in a protic solvent;
(b) adding the trialkylsilyl halogenide;
(c) allowing crystals to form and;
(d) collecting the crystals formed.

3. The process of claim 2 wherein in step (a) the protic solvent is a compound comprising a hydroxyl group or a carboxyl group, in particular wherein the protic solvent is an aromatic or aliphatic alcohol, a silanol, a ketone capable of enolization, or an aromatic or aliphatic carbonic acid, more particularly wherein the protic solvent is an C1-C6 alkyl alcohol, formic acid or acetic acid.

4. The process of claims 2 or 3 wherein in step (b) the trialkylsilyl halogenide is trimethylsilyl chloride, trimethylsilyl bromide or trimethylsilyl iodide, in particular trimethylsilylchloride.

5. The process of claim 1, which process comprises the steps:
(a) dissolving or suspending the organic amine in an aprotic solvent;
(b) adding at least one equivalent of a protic solvent;
(c) adding the trialkylsilyl halogenide;
(d) allowing crystals to form; and
(e) collecting the crystals formed.

6. The process of claim 5 wherein in step (b) the protic solvent is a compound comprising a hydroxyl group or a carboxyl group, in particular wherein the protic solvent is an aromatic or aliphatic alcohol, a silanol a ketone capable of enolization or an aromatic or aliphatic carbonic acid, more particularly wherein the protic solvent is an C1-C6 alkyl alcohol, formic acid or acetic acid.

7. The process of claims 5 or 6 wherein in step (c) the trialkylsilyl halogenide is trimethylsilyl chloride, trimethylsilyl bromide or trimethylsilyl iodide, in particular trimethylsilylchloride.

8. The process of claim 1, which process comprises the steps:
(a) dissolving, suspending or generating the acid addition salt of the organic amine in a solvent;
(b) adding the trialkylsilyl halogenide;
(c) allowing crystals to form; and
(d) collecting the crystals formed.

9. The process of claim 8 wherein in step (a) the acid addition salt of the organic amine is generated in situ by adding an acid, which conjugated acid is weaker than hydrochloric acid and preferably is an organic acid, to a solution or slurry of the amine.

10. The process of claims 8 to 9 wherein in step (a) the organic acid is selected from substituted or unsubstituted alkanoic acids, aromatic carboxylic acids, dicarboxylic acids or citric acid.

11. The process of claim 10, wherein the organic acid is acetic acid.

12. The process of any one of claims 1 to 10, wherein the organic amine is a pharmaceutically active compound, in particular a drug for humans.

13. The process of claim 12 wherein the drug comprises a primary, secondary, tertiary or quaternary amino group, in particular wherein the drug is selected from the group consisting of an antidepressant - in particular the serotonin reuptake inhibitor Sertraline, Duloxetine, Venlafaxine or Citalopram, a nootropic agent, in particular Donepezil, an antipsychotic agent, in particular the neuroleptic Aripiprazole, a muscle relaxant, in particular the antispasmodic agent Memantine, an immunosuppressant, in particular Mycophenolate Mofetil, an antifungal, in particular Terbinafine, an antibacterial, in particular a Quinolone as for example Moxifloxacin or the oxazolidinone Linezolid, a calcimimetic agent, in particular Cinacalcet, a Dopamine agonist, in particular the D2-receptor agonist Pramipexole, an antiobesity agent, in particular Rimonabant, an antithrombotic, in particular Clopidogrel and Prasugrel, an antiosteoporotic, in particular Raloxifene, an antispasmodic, in particular Darifenacin, an agent for treatment of male erectile dysfunction, in particular Vardenafil, an antidiabetic, in particular the DPP-IV inhibitor Sitagliptin, and an antineoplastic, in particular Erlotinib.

14. Use of a trialkylsilylhalogenide for the preparation of a crystalline hydrohalide of an organic amine.

15. Use according to claim 14, wherein the crystalline hydrohalide of an organic amine is one desired hydrohalide among several existing hydrohalides of said organic amine.

16. Use according to claims 14 or 15 for the preparation of an anhydrous crystalline hydrohalide of an organic amine.

17. Use according to claims 14 to 16 for the preparation of a solvate of a crystalline hydrohalide of an organic amine.

## Patentansprüche

1. Verfahren zur Herstellung eines kristallinen Hydrohalogenids eines organischen Amins, wobei ein Trialkylsilylhalogenid zu dem organischen Amin in einem Lösemittel zugegeben wird, wobei das organische Amin in Form der freien Base oder eines Säureadditionssalzes vorliegt, wobei, wenn das organische Amin in Form eines Säureadditionssalzes vorliegt, die konjugierte Säure des Säureadditionssalzes schwächer als die Halogenwasserstoffsäure ist.

2. Verfahren nach Anspruch 1, wobei das Verfahren die folgenden Stufen umfasst:
a) Auflösen oder Suspendieren des organischen Amins in einem protischen Lösemittel;
b) Zugeben des Trialkylsilylhalogenids;
c) Zulassen, dass Kristalle gebildet werden; und
d) Sammeln der gebildeten Kristalle.

3. Verfahren nach Anspruch 2, wobei in Stufe a) das protische Lösemittel eine Verbindung ist, die eine Hydroxylgruppe oder eine Carboxylgruppe umfasst, wobei insbesondere das protische Lösemittel ein aromatischer oder aliphatischer Alkohol, ein Silanol, ein Keton mit der Fähigkeit zur Enolisierung oder eine aromatische oder aliphatische Carbonsäure ist, wobei stärker bevorzugt das protische Lösemittel ein C1-C6-Alkylalkohol, Ameisensäure oder Essigsäure ist.

4. Verfahren nach den Ansprüchen 2 oder 3, wobei in Stufe b) das Trialkylsilylhalogenid Trimethylsilylchlorid, Trimethylsilylbromid oder Trimethylsilyliodid, insbesondere Trimethylsilylchlorid ist.

5. Verfahren nach Anspruch 1, wobei das Verfahren die folgenden Stufen umfasst:
a) Auflösen oder Suspendieren des organischen Amins in einem aprotischen Lösemittel;
b) Zugeben mindestens eines Equivalents eines protischen Lösemittels;
c) Zugeben des Trialkylsilylhalogenids;
d) Zulassen, dass Kristalle gebildet werden; und
e) Sammeln der gebildeten Kristalle.

6. Verfahren nach Anspruch 5, wobei in Stufe b) das protische Lösemittel eine Verbindung ist, die eine Hydroxylgruppe oder eine Carboxylgruppe umfasst, wobei insbesondere das protische Lösemittel ein aromatischer oder aliphatischer Alkohol, ein Silanol, ein Keton mit der Fähigkeit zur Enolisierung oder eine aromatische oder aliphatische Carbonsäure ist, wobei stärker bevorzugt das protische Lösemittel ein C1-C6-Alkylalkohol, Ameisensäure oder Essigsäure ist.

7. Verfahren nach den Ansprüchen 5 oder 6, wobei in Stufe c) das Trialkylsilylhalogenid Trimethylsilylchlorid, Trimethylsilylbromid oder Trimethylsilyliodid, insbesondere Trimethylsilylchlorid ist.

8. Verfahren nach Anspruch 1, wobei das Verfahren die folgenden Stufen umfasst:
a) Auflösen, Suspendieren oder Erzeugen des Säureadditionssalzes des organischen Amins in einem Lösemittel;
b) Zugeben des Trialkylsilylhalogenids;
c) Zulassen, dass Kristalle gebildet werden; und
d) Sammeln der gebildeten Kristalle.

9. Verfahren nach Anspruch 8, wobei in Stufe a) das Säureadditionssalz des organischen Amins in situ durch Zugeben einer Säure, wobei die konjugierte Säure schwächer als Salzsäure ist und vorzugsweise eine organische Säure ist, zu einer Lösung oder Aufschlämmung des Amins erzeugt wird.

10. Verfahren nach den Ansprüchen 8 oder 9, wobei die Stufe a) die organische Säure aus substituierten oder nicht substituierten Alkansäuren, aromatischen Carbonsäuren, Dicarbonsäuren oder Zitronensäure ausgewählt ist.

11. Verfahren nach Anspruch 10, wobei die organische Säure Essigsäure ist.

12. Verfahren nach einem der Ansprüche 1 bis 10, wobei das organische Amin eine pharmazeutisch aktive Verbindung, insbesondere ein Wirkstoff für Menschen ist.

13. Verfahren nach Anspruch 12, wobei der Wirkstoff eine primäre, sekundäre, tertiäre oder quartäre Aminogruppe umfasst, wobei insbesondere der Wirkstoff aus der Gruppe ausgewählt ist, die aus einem Antidepressivum - insbesondere dem Serotonin-Wiederaufnahmehemmer Sertralin, Duloxetin, Venlafaxin oder Citalopram, einem nootropen Mittel, insbesondere Donepezil, einem Antipsychotikum, insbesondere dem Neuroleptikum Aripiprazol, einen Muskelrelaxans, insbesondere dem Antispasmodikum Memantin, einem Immunosuppressivum, insbesondere Mycophenolat Mofetil, einem Antipilzmittel, insbesondere Terbinafin, einem antibakteriellen Mittel, insbesondere einem Chinolon wie beispielsweise Moxifloxacin oder dem Oxazolidinon Linezolid, einem calcimimetischen Mittel, insbesondere Cinacalcet, einem Dopaminagonisten, insbesondere dem D2-Rezeptor-Agonisten Pramipexol, einem gegen Fettleibigkeit gerichteten Mittel, insbesondere Rimonabant, einem Antithrombotikum, insbesondere Clopidogrel und Prasugrel, einem Antiosteoporotikum, insbesondere Raloxifen, einem Antispasmodikum, insbesondere Darifenacin, einem Mittel zur Behandlung männlicher erektiler Dysfunktion, insbesondere Vardenafil, einem Antidiabetikum, insbesondere dem DPP-IV-Inhibitor Sitagliptin, und einem Antineoplastikum, insbesondere Erlotinib, besteht.

14. Verwendung eines Trialkylsilylhalogenids zur Herstellung eines kristallinen Hydrohalogenids eines organischen Amins.

15. Verwendung nach Anspruch 14, wobei das kristalline Hydrohalogenid eines organischen Amins ein gewünschtes Hydrohalogenid unter mehreren existierenden Hydrohalogeniden des organischen Amins ist.

16. Verwendung nach den Ansprüchen 14 oder 15 zur Herstellung eines wasserfreien kristallinen Hydrohalogenids eines organischen Amins.

17. Verwendung nach den Ansprüchen 14 bis 16 zur Herstellung eines Solvats eines kristallinen Hydrohalogenids eines organischen Amins.

## Revendications

1. Procédé pour la préparation d'un hydrohalogénure cristallin d'une amine organique dans lequel un halogénure de trialkylsilyle est ajouté à l'amine organique dans un solvant, cette amine organique étant sous forme de base libre ou d'un sel d'addition d'acide, dans lequel lorsque l'amine organique est sous la forme d'un sel d'addition d'acide, l'acide conjugué du sel d'addition d'acide est plus faible que l'acide hydrohalogénique.

2. Le procédé selon la revendication 1, ce procédé comprenant les étapes de :
a. Dissolution ou suspension de l'amine organique dans un solvant protique ;
b. Addition de l'halogénure de trialkylsilyle ;
c. Formation de cristaux et ;
d. Récupération des cristaux formés.

3. Le procédé selon la revendication 2 dans lequel à l'étape (a), le solvant protique est un composé comprenant un groupe hydroxyle ou un groupe carboxyle, en particulier dans lequel le solvant protique est un alcool aromatique ou aliphatique, un silanol, une cétone capable d'énolisation ou un acide carbonique aromatique ou aliphatique, en particulier dans lequel le solvant protique est un alcool alkylique en C₁-C₆, l'acide formique ou l'acide acétique.

4. Le procédé de la revendication 2 ou 3 dans lequel, à l'étape (b), l'halogénure de trialkylsilyle est le chlorure de triméthylsilyle, le bromure de triméthylsilyle ou l'iodure de triméthylsilyle en particulier le chlorure de triméthylsilyle.

5. Le procédé selon la revendication 1, ce procédé comprenant les étapes de :
a. Dissolution ou suspension de l'amine organique dans un solvant aprotique ;
b. Addition d'au moins un équivalent d'un solvant protique ;
c. Addition d'un halogénure de trialkylsilyle ;
d. Formation de cristaux ; et
e. Récupération des cristaux formés.

6. Le procédé selon la revendication 5 dans lequel, à l'étape (b), le solvant protique est un alcool aromatique ou aliphatique, un silanol, une cétone capable d'énolisation ou un acide carbonique aromatique ou aliphatique, en particulier dans lequel le solvant protique est un alcool alkylique en C₁-C₆, l'acide formique ou l'acide acétique.

7. Le procédé selon la revendication 5 ou 6 dans lequel, à l'étape (c), l'halogénure de trialkylsilyle est le chlorure de triméthylsilyle, le bromure de triméthylsilyle ou l'iodure de triméthylsilyle, en particulier le chlorure de triméthylsilyle.

8. Le procédé selon la revendication 1, ce procédé comprenant les étapes de :
a. Dissolution, suspension ou formation du sel d'addition d'acide de l'amine organique dans un solvant ;
b. Addition de l'halogénure de trialkylsilyle ;
c. Formation de cristaux ; et
d. Récupération des cristaux formés.

9. Le procédé selon la revendication 8 dans lequel, à l'étape (b), le sel d'addition d'acide de l'amine organique est généré *in situ* par ajout d'un acide, cet acide conjugué étant plus faible que l'acide chlorhydrique et étant de préférence un acide organique, à une solution ou à une boue de l'amine.

10. Le procédé selon les revendications 8 à 9 dans lequel, à l'étape (a), l'acide organique est choisi parmi des acides alkanoïques substitués ou non substitués, des acides carboxyliques aromatiques, des acides dicarboxyliques ou l'acide citrique.

11. Le procédé selon la revendication 10, dans lequel l'acide organique est l'acide acétique.

12. Le procédé selon l'une des revendications 1 à 10, dans lequel l'amine organique est un composé pharmaceutiquement actif, en particulier un médicament pour humains.

13. Le procédé selon la revendication 12, dans lequel le médicament comprend un groupe amino primaire, secondaire, tertiaire ou quaternaire, en particulier dans lequel le médicament est choisi parmi le groupe formé par un antidépresseur - en particulier l'inhibiteur de recapture de la sérotonine Sertraline, Duloxetine, Venlafaxine ou Citalopram, un agent nootrope, en particulier Donepezil, un agent antipsychotique, en particulier le neuroleptique Aripiprazole, un myorelaxant, en particulier l'agent antispasmodique Memantine, un immunosuppresseur, en particulier Mycophenolate Mofetil, un antifongique en particulier Terbinafine, un antibactérien, en particulier une Quinolone comme par exemple Moxifloxacine, en particulier l'agoniste du récepteur D2 Pramipexole, un antiobésité en particulier Rimonabant, un antithrombotique, en particulier Clopidogrel et Prasugrel, un antiostéoporose, en particulier Raloxifène un antispamodique, en particulier Darifenacine, un agent pour le traitement du dysfonctionnement érectile chez le mâle, en particulier Vardenafil, un antidiabétique, en particulier l'inhibiteur de DDP-IV Sitagliptine et un antinéoplasique, en particulier Erlotinib.

14. Utilisation d'un halogénure de trialkylsilyle pour la préparation d'un hydrohalogénure d'une amine organique.

15. Utilisation selon la revendication 14 dans laquelle l'hydrohalogénure cristallin d'une amine organique est un hydrohalogénure désiré parmi plusieurs hydrohalogénures de ladite amine organique.

16. Utilisation selon la revendication 14 ou 15 pour la préparation d'un hydrohalogénure cristallin anhydre d'une amine organique.

17. Utilisation selon les revendications 14 à 16 pour la préparation d'un solvate d'un hydrohalogénure cristallin d'une amine organique.
